(19) Europäisches Patentamt
European Patent Office
Office européen des brevets

(11) EP 1 532 141 B1

(12) # EUROPÄISCHE PATENTSCHRIFT

(45) Veröffentlichungstag und Bekanntmachung des Hinweises auf die Patenterteilung:
**13.10.2010 Patentblatt 2010/41**

(21) Anmeldenummer: **03794933.6**

(22) Anmeldetag: **25.08.2003**

(51) Int Cl.:
***C07D 413/04*** (2006.01) ***C07D 413/14*** (2006.01)
***A61K 31/4245*** (2006.01) ***A61K 31/4439*** (2006.01)
***A61K 31/4725*** (2006.01) ***A61P 25/24*** (2006.01)

(86) Internationale Anmeldenummer:
**PCT/EP2003/009390**

(87) Internationale Veröffentlichungsnummer:
**WO 2004/024725 (25.03.2004 Gazette 2004/13)**

(54) **2-PYRROLIDIN-2-YL-[1,3,4] -OXADIAZOL-DERIVATE UND IHRE VERWENDUNG ALS ANTIDEPRESSIVA**

2-PYRROLIDIN-2-YL- [1,3,4]-OXADIAZOLE DERIVATIVES AND THE USE OF THE SAME AS ANTIDEPRESSANTS

DERIVES DE 2-PYRROLIDIN-2-YL- [1,3,4]-OXADIAZOLE ET LEUR UTILISATION EN TANT QU'ANTIDEPRESSEURS

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **30.08.2002 DE 10240818**

(43) Veröffentlichungstag der Anmeldung:
**25.05.2005 Patentblatt 2005/21**

(73) Patentinhaber: **Grünenthal GmbH**
**52078 Aachen (DE)**

(72) Erfinder:
- **HENNIES, Hagen-Heinrich**
**52152 Simmerath (DE)**
- **BUSCHMANN, Helmut**
**E-08950 Esplugues de Llobregat (ES)**
- **Die andere Erfinder haben auf ihre Nennung verzichtet.**

(74) Vertreter: **Bülle, Jan et al**
**Kutzenberger & Wolff**
**Patentanwaltssozietät**
**Theodor-Heuss-Ring 23**
**50668 Köln (DE)**

(56) Entgegenhaltungen:
**WO-A-01/04116 WO-A-02/02554**
**US-A- 3 912 747 US-A- 4 784 998**

EP 1 532 141 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft substituierte 2-Pyrrolidin-2-yl-[1,3,4]-oxadiazol - Derivate, Verfahren zu deren Herstellung, Arzneimittel enthaltend diese Verbindungen sowie die Verwendung dieser Stoffe zur Herstellung von Arzneimitteln, vorzugsweise zur Behandlung von Depressionen, und Verfahren zur Behandlung von Depressionen.

Depression ist eine Störung der Affektivität, bei der ein depressives Syndrom im Vordergrund steht, wobei depressiv mit Verstimmung verbunden bzw. traurig gestimmt heißt. Die zur Therapie verwendeten Antidepressiva sind auch wichtige Adjuvantien für die Schmerztherapie (van Schayck et al., MMP 1998, 21. Jahrgang, Heft 10, 304-313; Jung et al., J. Gen. Intern. Med. 1997, 12/6, 384-389; Onghena and Van Houdenhove, Pain 1992, 49, 205-219; Feuerstein, Der Schmerz 1997, 11, 213-226; Rowbotham, The Pain Medicine Journal Club 1997, 3/3, 119-122), insbesondere bei chronischen Schmerzzuständen, da die andauernde Schmerzbelastung zu einer depressiven Verstimmung der Patienten führen kann. Dies ist besonders häufig bei Krebs-Schmerzpatienten der Fall (Berard, Int. Med. J. 1996, 3/4, 257-259). Da es bisher keine Schmerzmittel mit einer klinisch relevanten antidepressiven Wirkkomponenten gibt, müssen die Antidepressiva als Zusatzmedikation zur Analgetika-Gabe hinzugefügt werden. Da chronische Schmerzpatienten häufig eine Vielzahl von verschiedenen Medikamenten benötigen, führt die zusätzliche Gabe des Antidepressivums zu einer weiteren Belastung des Organismus. Aus diesem Grund und zur Verbesserung der Compliance wäre eine analgetisch wirksame Substanz mit einer antidepressiven Wirkkomponente von besonderem Vorteil.

Grundlage der antidepressiven Wirkung ist die Wiederaufnahmehemmung von Serotonin.

Verschiedene substituierte 2-Pyrrolidin-2-yl-[1,3,4]-oxadiazol - Derivate sind aus der Literatur bekannt. Ihnen allen ist gemeinsam, dass sie für die Therapie von neuronalen Erkrankungen eingesetzt werden.

Eine Synthese für substituierte 2-Pyrrolidin-2-yl-[1,3,4]-oxadiazol - Derivate wurde bereits beschrieben durch Borg et al. (J. Org. Chem. 1995, 60, 3112-3120), wobei als Ausgangsmaterial natürliche Aminosäuren dienen und eine Dehydrierung von Diazylhydrazinen erfolgt, und Brain et al. (Synlett 2001, No. 3, 382-384), wobei die Cyclodehydrierung von 1,2-Diacylhydrazinen in der Mikrowelle mit einem Polystyrengestützten Dehydrierungsagenten durchgeführt wird. Bei diesen Synthesen handelt es sich um Festphasensynthesen.

Eine weitere Möglichkeit zur Synthese von Pyrrolidin- oder Piperidinderivaten zur Behandlung und Prävention von neuronalen Erkrankungen wurde in WO 01/04116 beschrieben.

JP 2001247569 beschreibt ebenfalls die Herstellung von Pyrrolidin- oder Piperidinderivaten und deren Verwendung für die Prophylaxe und/oder Behandlung von Erkrankungen, die von Verletzungen der Nerven oder Neurodegenerationen begleitet sind.

US 3912747 offenbart 2-Hydroxymethyl-1,3,4-oxadiazolderivate, die sich unter anderem zur Behandlung von Schmerz und Depressionen eignen.

Die der Erfindung zugrundeliegende Aufgabe bestand in dem zur Verfügung stellen einer neuen Strukturklasse analgetisch wirksamer Substanzen, die sich darüber hinaus insbesondere zur Behandlung von Depressionen und/oder zur Anxiolyse eignen.

Überraschenderweise wurde nun gefunden, dass substituierte 2-Pyrrolidin-2-yl-[1,3,4]-oxadiazol - Derivate der allgemeinen Formel I eine ausgeprägte antidepressive und auch analgetische Wirkung aufweisen.

Gegenstand der Erfindung sind daher substituierte 2-Pyrrolidin-2-yl-[1,3,4]-oxadiazol-Derivate der allgemeinen Formel I,

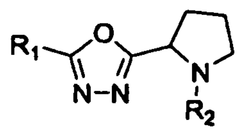

I

worin

$R_1$ Aryl oder Heteroaryl bedeutet,

$R_2$ H, $SO_2R^3$ oder $COR^4$ bedeutet; wobei

$R^3$ und $R^4$ unabhängig voneinander $C_{1-10}$-Alkyl, $C_{3-10}$-Cycloalkyl, ($C_{1-6}$-Alkyl)-$C_{3-10}$- Cycloalkyl, Aryl, ($C_{1-6}$-Alkyl)-Aryl, Heterocyclyl, Reste von Carbonsäureestem mit 3-10 C-Atomen, Dimethylamid oder $NR^5R^6$ bedeuten, wobei

$R^5$ und $R^6$ unabhängig voneinander H, Benzyl oder Phenyl bedeuten, wobei Benzyl oder Phenyl jeweils einfach oder mehrfach gleich oder verschieden substituiert ist mit F, Cl, O-Alkyl, CN, $CF_3$ oder $OCF_3$;

wobei der Begriff "Alkyl" acyclische, gesättigte oder ungesättigte, verzweigte oder geradkettige, unsubstituierte

oder einfach oder mehrfach substituierte Kohlenwasserstoffreste umfasst, wobei auch Reste umfasst sind, deren C-Kette durch ein oder mehrere der Heteroatome N, O oder S unterbrochen ist, und der Begriff "Cycloalkyl" cyclische Kohlenwasserstoffreste umfasst, die gesättigt oder ungesättigt, unsubstituiert oder einfach oder mehrfach substituert bzw. gegebenfalls benzokondensiert sein können,

wobei man unter dem Begriff "substituiert" in Bezug auf "Alkyl und "Cycloalkyl" die einfache oder mehrfache Substitution von einem oder mehreren Wasserstoffatomen durch F, Cl, Br, I, -CN, $NH_2$, NH-Alkyl, NH-Aryl, NH- Alkyl-Aryl, NH-Heterocyclyl, $N(Alkyl)_2$, $N(Alkyl\text{-}Aryl)_2$, N-Alkyl-N-Aryl, $NO_2$, OH, Ketogruppe, O-Alkyl, O-Aryl, O-Alkyl-Aryl, C(=O)$C_{1-6}$-Alkyl, C(=O)Aryl, C(=O)$C_{1-6}$-Alkyl-Aryl, C(=O)-Heterocyclyl, $CO_2H$, $CO_2$-Alkyl, $CO_2$-Alkyl-Aryl, C(=O)$NH_2$, C(=O)NH-Alkyl, C(=O)NHAryl, C(=O)NH- Heterocyclyl, C(=O)N(Alkyl)$_2$, C(=O)N(Alkyl-Aryl)$_2$, Cycloalkyl, Aryl oder Heterocyclyl, versteht.

Vorzugsweise bedeuten in Verbindungen nach Formel I

| | |
|---|---|
| $R_1$ | Aryl oder Heteroaryl, wobei Aryl für Phenyl unsubstituiert oder substituiert mit F, Cl, O-Alkyl oder Phenylund Heteroaryl für Pyridinyl oder Thienyl steht, |
| $R_2$ | H, $SO_2R^3$ oder $COR^4$, wobei |
| $R^3$ und $R^4$ | unabhängig voneinander $C_{1-10}$-Alkyl, $C_{3-10}$-Cycloalkyl, ($C_{1-6}$-Alkyl)- $C_{3-10}$-Cycloalkyl, Aryl, ($C_{1-6}$-Alkyl)-Aryl, Heterocyclyl, Reste von Carbonsäureestem mit 3-10 C-Atomen, Dimethylamid oder $NR^5R^6$ bedeuten, wobei |
| $C_{1-10}$-Alkyl | für Methyl, Propyl, Butyl, Butenyl, Isobutyl, Pentyl, Pent-3-yl, Hept-3- yl, Hept-4-yl, 2,2-Dimethylpropyl, $CH_2OCH_3$ oder $CH_2O(CH_2)_2OCH_3$ steht, |
| $C_{3-10}$-Cycloalkyl für | Cyclopropyl, Cyclobutyl, Cyclopentyl, Adamantan-1-yl oder 2-Phenyl-cyclopropyl steht, |
| ($C_{1-6}$-Alkyl)-$C_{3-10}$-Cycloalkyl für | $CH_2$-Cyclopentyl, $(CH_2)_2$-Cyclopentyl oder 7,7-Dimethyl-1-methyl-bicyclo[2.2.1]heptan-2-on steht, |
| Aryl für | Phenyl, Benzyl oder Naphthyl unsubstituiert, einfach substituiert oder mehrfach gleich oder verschieden mit Phenyl, $NO_2$, $C_{1-6}$-Alkyl, dessen C-Atomkette ggf. durch ein oder mehrere der Heteroatome N, O oder S, vorzugsweise durch O, unterbrochen ist, O-Alkyl, $CO_2$-Alkyl, C(=O)$C_{1-6}$- Alkyl, $CH_2OC(=O)C_6H_5$, F, Cl, Br, $N(CH_3)_2$, $OCF_3$, $CF_3$, $SCHF_2$, $SCF_3$ oder $(C{=}O)CH_3$ steht, |
| ($C_{1-6}$-Alkyl)-Aryl für | 3,4-Dimethoxyphenyl-$CH_2$, 4-Chlorphenoxy-$CH_2$, Phenyl- CH=CH, Benzyl-$OCH_2$, Phenyl-$(CH_2)_2$, 2-Bromphenyl-$CH_2$, 1-Phenylpropyl, 2-Chlorphenyl-CH=CH, 3-Trifluormethyl-phenyl-CH=CH, Phenoxy-$CH_2$, Phenoxy-$(CH_2)_3$ oder Phenoxy-CH($CH_3$) steht, |
| Heterocyclyl für | Pyridinyl, Isoxazol, Thienyl, Furanyl, Triazol, Benzooxadiazol, Thiadiazol, Pyrazol oder Isochinolin, unsubstituiert, einfach substituiert oder mehrfach gleich oder verschieden mit Cl, $C_{1-6}$-Alkyl, Phenyl, welches wiederum unsubstituiert oder einfach oder mehrfach gleich oder verschieden mit Cl oder $C_{1-6}$-Alkyl substituiert ist, $CF_3$, C(=O)$CF_3$ oder $SCH_3$ steht, |

die Reste von Carbonsäureestem mit 3-10 C-Atomen für
$CH_3OC(=O)CH_2$
$CH_3OC(=O)(CH_2)_3$
$CH_3CH_2OC(=O)CH_2$
$CH_3CH_2OC(=O)(CH_2)_2$
$CH_3C(=O)OCH_2$
$CH_3C(=O)OC(CH_3)_2$ oder
$CH_3C(=O)OCH(C_6H_5)$ stehen
und

$R^5$ und $R^6$ unabhängig voneinander H, Benzyl oder Phenyl bedeuten, wobei Benzyl oder Phenyl, jeweils einfach oder mehrfach gleich oder verschieden substituiert ist mit F, Cl, O-Alkyl, CN, $CF_3$ oder $OCF_3$.

Besonders bevorzugt sind Verbindungen nach Formel I, in denen

$R_1$ Phenyl, Bisphenyl-4-yl, 3-Methoxyphenyl, 4-Chlorphenyl, 2-Fluorphenyl, Pyridin-3-yl, Pyridin-4-yl oder Thiophen-2-yl bedeutet,

$R_2$ H, $SO_2R^3$ oder $COR^4$ bedeutet, wobei

$R^3$ und $R^4$ unabhängig voneinander $CH_3CH_2OCO(CH_2)_2$- , 2,4-Dimethoxy- phenyl, 2-Chlor-pyridin-3-yl, 2-Chlor-pyridin-4-yl, 7,7-Dimethyl-1-methyl- bicyclo[2.2.1]heptan-2-on, 3-Dimethyl-amino-phenyl, 3,4-Dimethoxy-phenyl, 2,5-Dimethoxy-phenyl, 4-Chlor-phenyl, 4,5-Dichlor-thiophen-2-yl, 2,4,6-Trimethylphenyl, 4-Chlor-phenoxy-methyl, $C_6H_5CH$=CH-, 5-Methyl-2-phenyl-2H-[1,2,3]triazol-4-yl, 2,4-Difluorphenyl, 2,6- Difluor-phenyl, 4-Brom-phenyl, 4-Ethoxy-phenyl, 4-Trifluormethoxy- phenyl, 2,5-Bis-trifluormethyl-phenyl, 1-Phenyl-5-propyl-1H-pyrazol-4-yl, 3-Methoxy-phenyl, 2-Methyl-6-trifluormethyl-pyridin-3-yl, 4- Trifuormethylsulfanyl-phenyl, 2,2,2-Trifluor-1-3,4-dihydro-1H- isochinolin-2-yl-ethanon oder $NR^5R^6$ bedeuten, wobei

$R^5$ und $R^6$ unabhängig voneinander H oder 3-Methoxyphenyl, 4-Methoxyphenyl, 4-Ethoxyphenyl, 2,5-Dimethoxy-phenyl, 2,5-Dichlorphenyl, 2,5-Difluorphenyl, 4-Fluorbenzyl, 4-Chlor-3- trifluormethyl-phenyl, 4-Trifuormethoxy-phenyl oder 3-Cyanophenyl bedeuten.

Insbesondere bevorzugt sind folgende substituierte 2-Pyrrolidin-2-yl-[1,3,4]-oxadiazol - Derivate:

4-Oxo-4-[2-(5-pyridin-4-yl-[1,3,4]oxadiazol-2-yl)-pyrrolidin-1-yl]-buttersäureethylester

(2,4-Dimethoxy-phenyl)-[2-(5-pyridin-4-yl-[1,3,4]oxadiazol-2-yl)-pyrrolidin-1-yl]-methanon

(2-Chlor-pyridin-3-yl)-[2-(5-pyridin-4-yl-[1,3,4]oxadiazol-2-yl)-pyrrolidin-1-yl]-methanon

7,7-Dimethyl-1-[2-(5-pyridin-4-yl-[1,3,4]oxadiazol-2-yl)-pyrrolidin-1-sulfonylmethyl]-bicyclo[2.2.1]heptan-2-on

(3-Dimethylamino-phenyl)-[2-(5-pyridin-3-yl-[1,3,4]oxadiazol-2-yl)-pyrrolidin-1-yl]-methanon

2-(3,4-Dimethoxy-phenyl)-1-[2-(5-pyridin-4-yl-[1,3,4]oxadiazol-2-yl)-pyrrolidin-1-yl]-ethanon

[2-(5-Biphenyl-4-yl-[1,3,4]oxadiazol-2-yl)-pyrrotidin-1-yl]-(4-chlor-phenyl)-methanon

4-{5-[1-(4,5-Dichlor-thiophene-2-sulfonyl)-pyrrolidin-2-yl]-[1,3,4]oxadiazol-2-yl}-pyridin

3-{5-[1-(2,5-Dimethoxy-benzylsulfonyl)-pyrrolidin-2-yl]-[1,3,4]oxadiazol-2-yl}-pyridin

2-(2-Fluor-phenyl)-5-[1-(2,4,6-trimethyl-benzylsulfonyl)-pyrrolidin-2-yl]-[1,3,4]oxadiazol

2-(4-Chlor-phenoxy)-1-[2-(5-pyridin-4-yl-[1,3,4]oxadiazol-2-yl)-pyrrolidin-1-yl]-ethanon

3-Phenyl-1-[2-(5-phenyl-[1,3,4]oxadiazol-2-yl)-pyrrolidin-1-yl]-propenon

(5-Methyl-2-phenyl-2H-[1,2,3]triazol-4-yl)-[2-(5-pyridin-4-yl-[1,3,4]oxadiazol-2-yl)-pyrrolidin-1-yl]-methanon

3-{5-[1-(2,4-Difluor-benzylsulfonyl)-pyrrolidin-2-yl]-[1,3,4]oxadiazol-2-yl}-pyridin

(4-Brom-phenyl)-{2-[5-(3-methoxy-phenyl)-[1,3,4]oxadiazol-2-yl]-pyrrolidin-1-yl}-methanon

(2-Chlor-pyridin-4-yl)-{2-[5-(3-methoxy-phenyl)-[1,3,4]oxadiazol-2-yl]-pyrrolidin-1-yl}-methanon

(2,6-Difluor-phenyl)-[2-(5-pyridin-4-yl-[1,3,4]oxadiazol-2-yl)-pyrrolidin-1-yl]-methanon

(4-Ethoxy-phenyl)-[2-(5-phenyl-[1,3,4]oxadiazol-2-yl)-pyrrolidin-1-yl]-methanon

3-{5-[1-(4-Trifluormethoxy-benzylsulfonyl)-pyrrolidin-2-yl]-[1,3,4]oxadiazol-2-yl}-pyridin

(2,5-Bis-trifluormethyl-phenyl)-{2-[5-(4-chlor-phenyl)-[1,3,4]oxadiazol-2-yl]-pyrrolidin-1-yl}-methanon

(2,5-Bis-trifluormethyl-phenyl)-[2-(5-thiophen-2-yl-[1,3,4]oxadiazol-2-yl)-pyrrolidin-1-yl]-methanon

(1-Phenyl-5-propyl-1H-pyrazol-4-yl)-[2-(5-pyridin-3-yl-[1,3,4]oxadiazol-2-yl)-pyrrolidin-1-yl]-methanon

(2,3-Difluor-phenyl)-{2-[5-(3-methoxy-phenyl)-[1,3,4]oxadiazol-2-yl]-pyrrolidin-1-yl}-methanon

(2-Methyl-6-trifluormethyl-pyridin-3-yl)-[2-(5-phenyl-[1,3,4]oxadiazol-2-yl)-pyrrolidin-1-yl]-methanon

[2-(5-Phenyl-[1,3,4]oxadiazol-2-yl)-pyrrolidin-1-yl]-(4-trifluormethylsulfanyl-phenyl)-methanon

2,2,2-Trifluor-1-{7-[2-(5-pyridin-4-yl-[1,3,4]oxadiazol-2-yl)-pyrrolidin-1-sulfonyl]-3,4-dihydro-1H-isochinolin-2-yl}-ethanon

1-{2-[5-(3-Chlor-phenyl)-[1,3,4]oxadiazol-2-yl]-pyrrolidin-1-yl}-3-cyclopentyl-propan-1-on

1-{2-[5-(3-Chlor-phenyl)-[1,3,4]oxadiazol-2-yl]-pyrrolidin-1-yl}-pentan-1-on

Die Begriffe "Alkyl", "$C_{1-10}$-Alkyl", und "$C_{1-6}$Alkyl" umfassen im Sinne dieser Erfindung acyclische gesättigte oder ungesättigte Kohlenwasserstoffreste, die verzweigt oder geradkettig sowie unsubstituiert oder einfach substituiert oder mehrfach gleich oder verschieden substituiert sein können, mit (wie im Fall von $C_{1-10}$-Alkyl) 1 bis 10 (d.h. 1, 2, 3, 4, 5, 6, 7, 8, 9 oder 10) bzw. (wie im Fall von $C_{1-6}$-Alkyl) 1 bis 6 (d.h. 1, 2, 3, 4, 5 oder 6), d.h. $C_{1-10}$-, $C_{1-6}$-Alkanyle, $C_{2-10}$-, $C_{2-6}$-Alkenyle und $C_{2-10}$-, $C_{2-6}$-Alkinyle. Dabei weisen "Alkenyle" mindestens eine C-C-Doppelbindung und "Alkinyle" mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sek-Butyl, tert-Butyl, n-Pentyl, iso-Pentyl, neo-Pentyl, n-Hexyl, 2-Hexyl, n-Heptyl, n-Octyl, n-Nonyl, n-Decyl; Ethenyl (Vinyl), Ethinyl, Propenyl ($-CH_2CH=CH_2$, $-CH=CH-CH_3$, $-C(=CH_2)-CH_3$), Propinyl ($-CH_2-C{=}CH$, $-C{=}C-CH_3$), Butenyl, Butinyl, Pentenyl, Pentinyl, Hexenyl, Hexinyl, Octenyl und Octinyl umfaßt. Unter "Alkyl" im Sinne dieser Erfindung werden auch Reste verstanden, deren C-Atomkette ggf. durch ein oder mehrere der Heteroatome N, Ö oder S, vorzugsweise durch O, unterbrochen ist.

"$C_{3-10}$-Cycloalkyl" (bzw. "Cycloalkyl") bedeutet im Sinne dieser Erfindung cyclische gesättigte oder ungesättigte Kohlenwasserstoff-Reste mit 3, 4, 5, 6, 7. 8,9 oder 10 C-Atomen, wobei jeder der Reste unsubstituiert oder einfach substituiert oder mehrfach gleich oder verschieden substituiert und ggf. benzokondensiert sein kann. Ferner kann es sich um ein bi-, tri- oder polycyclisches Ringsystem handeln. Beispielhaft steht Cycloalkyl für Cyclopropyl, Cyclobutyl, Cyclopentyl, Cyclohexyl, Cycloheptanol und Cyclooctanyl sowie für Adamantyl und Bicyclo[2.2.1]heptyl.

Unter dem Ausdruck "Aryl" ist für die Zwecke der vorliegenden Erfindung ein Rest zu verstehen, der aus der Gruppe, die Phenyl, Naphthyl, Phenanthrenyl, Anthracenyl und Biphenyl umfaßt, ausgewählt ist und unsubstituiert oder einfach oder mehrfach gleich oder verschieden substituiert ist. Bevorzugt ist Aryl ein unsubstituiertes oder einfach substituiertes oder mehrfach gleich oder verschieden substituiertes Phenyl, 1-Naphthyl oder 2-Naphthyl.

Der Ausdruck "Heterocyclyl" steht für einen monocyclischen oder polycyclischen organischen Rest, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist. Beispiele für Heterocyclyl-Reste im Sinne dieser Erfindung sind monocyclische fünf-, sechs- oder siebengliedrige organische Reste mit 1 Heteroatom oder 2, 3, 4 oder 5 gleichen oder verschiedenen Heteroatomen, bei dem/denen es sich um Stickstoff, Sauerstoff und/oder Schwefel handelt, und deren benzokondensierte Analoga. Eine Untergruppe der Heterocyclyl-Reste bilden die "Heteroaryl"-Reste, bei denen es sich um solche Heterocyclyle handelt, in denen der mindestens eine Cyclus, der das/die Heteroatom/e enthält, heteroaromatisch ist. Jeder Heteroaryl-Rest kann unsubstituiert oder einfach substituiert oder mehrfach gleich oder verschieden substituiert vorliegen. Beispiele für Heterocyclyl-Reste im Sinne der vorliegenden Erfindung sind Pyrrolidinyl, Piperidinyl und Morpholinyl. Beispiele für Heteroaryl-Reste sind Pyrrolyl, Furanyl, Thienyl, Thiadiazolyl, Triazolyl, Isoxazolyl, Isochinolin, Pyrazolyl, Imidazolyl, Pyridazinyl, Pyrimidinyl, Pyrazinyl, Pyridinyl und Oxadiazolyl sowie deren benzokondensierte Analoga (z.B. Benzooxadiazolyl). Alle diese Reste können jeweils unsubstituiert oder substituiert vorlieben.

Die Ausdrücke "($C_{1-6}$-Alkyl)-$C_{3-10}$-Cycloalkyl" und "($C_{1-6}$-Alkyl)-Aryl" bedeuten für die Zwecke der vorliegenden Erfindung, dass der Cycloalkyl-, bzw. Aryl-Rest über eine $C_{1-6}$-Alkyl-Gruppe an die mit ihm substituierte Verbindung gebunden ist.

Im Zusammenhang mit "Alkyl", "Alkanyl", "Alkenyl", "Alkinyl" und "Cycloalkyl" versteht man unter dem Begriff "substituiert" im Sinne dieser Erfindung die einfache oder mehrfache Substitution von einem oder mehreren Wasserstoffatomen durch beispielsweise F, Cl, Br, I, -CN, $NH_2$, NH-Alkyl, NH-Aryl, NH-Alkyl-Aryl, NH-Heterocyclyl, $N(Alkyl)_2$, $N(Alkyl\text{-}Aryl)_2$, N-Alkyl-N-Aryl, $NO_2$, OH, Ketogruppe, O-Alkyl, O-Aryl, O-Alkyl-Aryl, $C(=O)C_{1-6}$-Alkyl, C(=O)Aryl, $C(=O)C_{1-6}$-Alkyl-Aryl, C(=O)-Heterocyclyl, $CO_2H$, $CO_2$-Alkyl, $CO_2$-Alkyl-Aryl, $C(=O)NH_2$, C(=O)NH-Alkyl, C(=O)NHAryl, C(=O)NH-Heterocyclyl, $C(=O)N(Alkyl)_2$, $C(=O)N(Alkyl\text{-}Aryl)_2$, Cycloalkyl, Aryl oder Heterocyclyl, wobei die mehrfache Substitution entweder an verschiedenen oder an gleichen Atomen mehrfach, z.B. zwei- oder dreifach, erfolgt. Die Mehrfachsubstitution kann mit dem gleichen oder mit verschiedenen Substituenten erfolgen. Möglich ist darüber hinaus auch die Substitution mit einem Sulfonamid.

In Bezug auf "Aryl", "Heterocyclyl" sowie "Heteroaryl" versteht man im Sinne dieser Erfindung unter "substituiert" die ein- oder mehrfache, z.B. zwei-, drei- oder vierfache, Substitution eines oder mehrerer Wasserstoffatome des Ringsystems durch einen geeigneten Substituenten. Soweit die Bedeutung dieser geeigneten Substituenten im Zusammenhang mit "Aryl", "Heterocyclyl" oder "Heteroaryl" nicht an anderer Stelle der Beschreibung oder in den Ansprüchen definiert ist, sind geeignete Substituenten F, Cl, Br, I, -CN, $NH_2$, NH-Alkyl, NH-Aryl, NH-Alkyl-Aryl, NH-Heterocyclyl, $N(Alkyl)_2$, $N(Alkyl\text{-}Aryl)_2$, $NO_2$, SH, S-Alkyl, OH, O-Alkyl, O-Cycloalkyl, O-Aryl, O-Alkyl-Aryl, O-Heterocyclyl, CHO, $C(=O)C_{1-6}$-Alkyl, $C(=O)CF_3$, C(=O)Aryl, $C(=O)\text{-}C_{1-6}$-Alkyl-Aryl, $CO_2H$, $CO_2$-Alkyl, -Alkyl-$CO_2$-Aryl, $C(=O)NH_2$, C(=O)NH-Alkyl, C(=O)NHAryl, C(=O)NH-Heterocyclyl, $C(=O)N(Alkyl)_2$, $SO_2NH_2$, $SO_3H$, $CF_3$, $CHF_2$, $CH_2F$, $OCF_3$, $OCHF_2$, $OCH_2F$, $SCF_3$, $SCHF_2$, Alkyl, Cycloalkyl, Aryl und/oder Heterocyclyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Besonders bevorzugte Substituenten für Aryl und Heterocyclyl sind $C_{1-6}$-Alkyl, F, Cl, Br, I, $CF_3$, OAlkyl, $OCF_3$, Phenyl, CN und/oder $NO_2$.

"Benzokondensiert" bedeutet für die Zwecke der vorliegenden Erfindung, dass ein Benzol-Ring an einen anderen Cyclus ankondensiert ist.

Ein weiterer Gegenstand der Erfindung ist ein Verfahren zur Herstellung von substituierten 2-Pyrrolidin-2-yl-[1,3,4]-oxadiazol - Derivaten der allgemeinen Formel I. Die erfindungsgemäßen Substanzen werden nach dem folgenden Syntheseschema hergestellt:

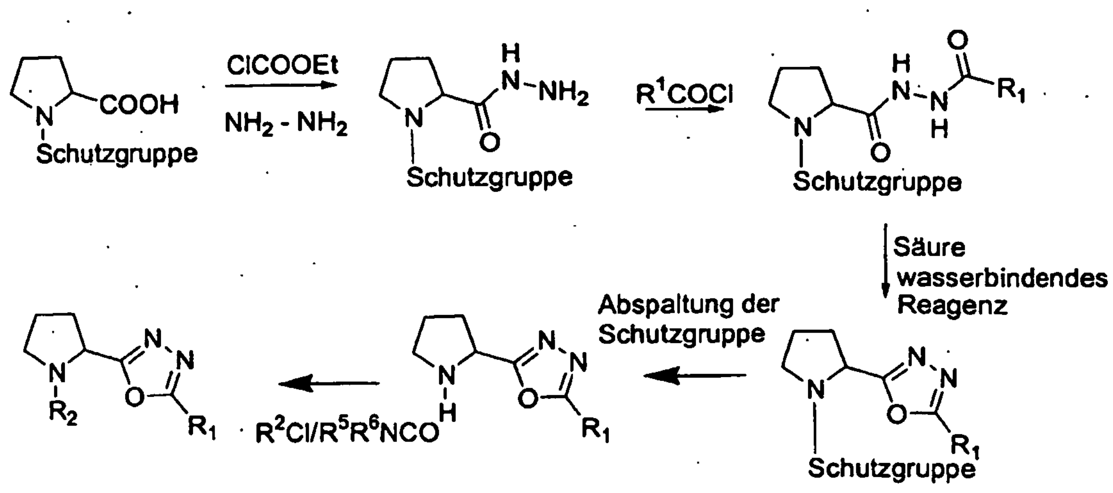


Zur Herstellung der erfindungsgemäßen substituierten 2-Pyrrolidin-2-yl-[1,3,4]-oxadiazol - Derivate wird Boc-Prolin mit Ethylchlorformat und Hydrazin in einem geeigneten Lösungsmittel, beispielsweise THF (Tetrahydrofuran), umgesetzt. Das resultierende Hydrazid wird mit einem Säurechlorid in einem geeigneten Lösungsmittel, beispielsweise THF, zu einem Diacylhydrazid umgesetzt. Für den Ringschluss wird das Diacylhydrazid mit einer Säure und einem wasserentziehenden Reagenz, beispielsweise $P_2O_5$, $CH_3SO_3H$, Pyridin und/oder $SOCl_2$ versetzt. Nach dem Ringschluss wird die Schutzgruppe abgespalten und das Produkt mit einem Säurechlorid oder einem Isocyanat umgesetzt und man erhält die erfindungsgemäßen Verbindungen der allgemeinen Formel I.

Die erfindungsgemäßen substituierten 2-Pyrrolidin-2-yl-[1,3,4]-oxadiazol - Derivate der allgemeinen Formeln I sind toxikologisch unbedenklich und eignen sich daher als pharmazeutische Wirkstoffe in Arzneimitteln.

Ein weiterer Gegenstand der vorliegenden Erfindung sind daher Arzneimittel, die wenigstens ein erfindungsgemäßes substituiertes 2-Pyrrolidin-2-yl-[1,3,4]-oxadiazol-Derivat der allgemeinen Formel I sowie gegebenenfalls physiologisch verträgliche Hilfsstoffe enthalten.

Vorzugsweise eignen sich die erfindungsgemäßen Arzneimittel zur Bekämpfung von Schmerzen und zur Behandlung oder Prophylaxe von Depressionen, Harninkontinenz, Diarrhöe, Pruritus, Alkohol-, Drogen und oder Medikamentenabhängigkeit, Übelkeit und Erbrechen, zur Anxiolyse, Vigilanzsteigerung und/oder Libidosteigerung.

Die Verwendung wenigstens eines substituierten 2-Pyrrolidin-2-yl-[1,3,4]-oxadiazol - Derivates der allgemeinen Formel I zur Herstellung eines Arzneimittels zur Bekämpfung von Schmerzen und zur Behandlung oder Prophylaxe von Depressionen, Harninkontinenz, Diarrhöe, Pruritus, Alkohol-, Drogen und oder Medikamentenabhängigkeit, Übelkeit und Erbrechen, zur Anxiolyse, Vigilanzsteigerung und/oder Libidosteigerung ist ebenfalls Gegenstand der vorliegenden Erfindung.

Die erfindungsgemäßen Arzneimittel können als flüssige, halbfeste oder feste Arzneiformen, beispielsweise in Form von Injektionslösungeh, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, vorliegen und.als solche auch verabreicht werden.

Neben wenigstens einem erfindungsgemäßen substituierten 2-Pyrrolidin-2-yl-[1,3,4]-oxadiazol - Derivat der allgemeinen Formel I enthalten die erfindungsgemäßen Arzneimittel üblicherweise weitere physiologisch verträgliche pharmazeutische Hilfsstoffe, die bevorzugt ausgewählt sind aus der Gruppe betstehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln; Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Formeln I auch verzögert freisetzen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen erfindungsgemäßen Verbindung der allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.05 bis 5 mg/kg Körpergewicht des Patienten wenigstens eines erfindungsgemäßen substituierten 2-Pyrrolidin-2-yl-[1,3,4]-oxadiazol - Derivates der allgemeinen Formel I appliziert.

Die erfindungsgemäßen Verbindungen fallen zwar unter die umfassende allgemeine Formel der Anmeldung JP 2001247569, sind aber dort an keiner Stelle expressis verbis genannt und gehören dort auch nicht zu den bevorzugten Verbindungen. Die dort beschriebenen Verbindungen sollen sich zur Verlängerung von Nervehfortsätzen und daher zur Behandlung und/oder Prävention von Krankheiten wie diabetische Nervenstörungen, Neuropathie, durchtrennte Nerven, nervenzerstörender Krankheiten wie ALS oder Multiple Sklerose, Alzheimer, Parkinson, Huntington Chorea oder Rükkenmarksverletzungen eignen.

Im Gegensatz dazu konnte für die erfindungsgemäßen analgetisch wirksamen Verbindungen eine deutliche antidepressive Wirkung nachgewiesen werden.

**Beispiele**

| Beispiel - Nr. | Erfindungsgemäße Verbindung |
|---|---|
| 1 | 4-{5-[1-(4-Nitro-benzylsulfonyl)-pyrrolidin-2-yl]-[1,3,4]oxadiazol-2-yl}-pyridin |
| 2 | 3-Oxo-3-[2-(5-pyridin-3-yl-[1,3,4]oxadiazol-2-yl)-pyrrolidin-1-yl]-propionsäureethylester |
| 3 | 4-{5-[1-(2,4,6-Trimethyl-benzylsulfonyl)-pyrrolidin-2-yl]-[1,3,4]oxadiazol-2-yl}-pyridin |
| 4 | 4-Oxo-4-[2-(5-pyridin-4-yl-[1,3,4]oxadiazol-2-yl)-pyrrolidin-1-yl]-buttersäureethylester |
| 5 | (2,4-Dimethoxy-phenyl)-[2-(5-pyridin-4-yl-[1,3,4]oxadiazol-2-yl)-pyrrolidin-1-yl]-methanon |
| 6 | (2-Chlor-pyridin-3-yl)-[2-(5-pyridin-4-yl-[1,3,4]oxadiazol-2-yl)-pyrrolidin-1-yl]-methanon |

(fortgesetzt)

| Beispiel - Nr. | Erfindungsgemäße Verbindung |
|---|---|
| 7 | 7,7-Dimethyl-1-[2-(5-pyridin-4-yl-[1,3,4]oxadiazol-2-yl)-pyrrolidin-1-sulfonylmethyl]-bicyclo[2.2.1] heptan-2-on |
| 8 | 1-[2-(5-Pyridin-4-yl-[1,3,4]oxadiazol-2-yl)-pyrrolidin-1-yl]-butan-1-on |
| 9 | [2-(5-Biphenyl-4-yl-[1,3,4]oxadiazol-2-yl)-pyrrolidin-1-yl]-(4-methoxy-phenyl)-methanon |
| 10 | 2-[1-(3-Chlor-4-fluor-benzylsulfonyl)-pyrrolidin-2-yl]-5-thiophen-2-yl-[1,3,4]oxadiazol |
| 11 | [3-(2,6-Dichlor-phenyl)-5-methyl-isoxazol-4-yl]-[2-(5-pyridin-4-yl-[1,3,4]oxadiazol-2-yl)-pyrrolidin-1-yl]-methanon |
| 12 | (3-Dimethylamino-phenyl)-[2-(5-pyridin-3-yl-[1,3,4]oxadiazol-2-yl)-pyrrolidin-1-yl]-methanon |
| 13 | 2-(3,4-Dimethoxy-phenyl)-1-[2-(5-pyridin-4-yl-[1,3,4]oxadiazol-2-yl)-pyrrolidin-1-yl]-ethanon |
| 14 | [2-(5-Biphenyl-4-yl-[1,3,4]oxadiazol-2-yl}-pyrrolidin-1-yl]-(4-chlor-phenyl)-methanon |
| 15 | 4-{5-[1-(4-Chlor-benzylsulfonyl)-pyrrolidin-2-yl]-[1,3,4]oxadiazol-2-yl}-pyridin |
| 16 | 4-{5-[1-(4-Methoxy-benzylsulfonyl)-pyrrolidin-2-yl]-[1,3,4]oxadiazol-2-yl}-pyridin |
| 17 | Isoxazol-5-yl-[2-(5-pyridin-3-yl-[1,3,4]oxadiazol-2-yl)-pyrrolidin-1-yl]-methanon |
| 18 | 4-{5-[1-(Butan-1-sulfonyl)-pyrrolidin-2-yl]-[1,3,4]oxadiazol-2-yl}-pyridin |
| 19 | 2-(2-Methoxy-ethoxy)-1-{2-[5-(3-methoxy-phenyl)-[1,3,4]oxadiazol-2-yl]-pyrrolidin-1-yl}-ethanon |
| 20 | Cyclobutyl-[2-(5-pyridin-3-yl-[1,3,4]oxadiazol-2-yl)-pyrrolidin-1-yl]-methanon |
| | |
| 22 | 4-{5-[1-(4,5-Dichlor-thiophene-2-sulfonyl)-pyrrolidin-2-yl]-[1,3,4]oxadiazol-2-yl}-pyridin |
| 23 | 1-[2-(5-Phenyl-[1,3,4]oxadiazol-2-yl)-pyrrolidin-1-yl]-ethanon |
| 24 | 1-{2-[5-(2-Fluor-phenyl)-[1,3,4]oxadiazol-2-yl]-pyrrolidin-1-yl}-2-methoxy-ethanon |
| 25 | (2-Chlor-pyridin-4-yl)-[2-(5-pyridin-3-yl-[1,3,4]oxadiazol-2-yl)-pyrrolidin-1-yl]-methanon |
| 26 | [2-(5-Pyridin-4-yl-[1,3,4]oxadiazol-2-yl)-pyrrolidin-1-yl]-(3,4,5-trimethoxy-phenyl)-methanon |
| 27 | (4-Brom-phenyl)-{2-[5-(4-chlor-phenyl)-[1,3,4]oxadiazol-2-yl]-pyrrolidin-1-yl}-methanon |
| 28 | 5-{2-[5-(2-Fluor-phenyl)-[1,3,4]oxadiazol-2-yl]-pyrrolidin-1-yl}-5-oxo-valeriansäuremethylester |
| 29 | [2-(5-Biphenyl-4-yl-[1,3,4]oxadiazol-2-yl)-pyrrolidin-1-yl]-[3-(2-chlor-phenyl)-5-methyl-isoxazol-4-yl]-methanon |
| 30 | 3-{5-[1-(2,5-Dimethoxy-benzylsulfonyl)-pyrrolidin-2-yl]-[1,3,4]oxadiazol-2-yl}-pyridin |
| 31 | (2-Methylsulfanyl-pyridin-3-yl)-[2-(5-pyridin-3-yl-[1,3,4]oxadiazol-2-yl)-pyrrolidin-1-yl]-methanon |
| 32 | 2-Phenyl-1-[2-(5-pyridin-4-yl-[1,3,4]oxadiazol-2-yl)-pyrrolidin-1-yl]-ethanon |
| 33 | 3-Oxo-3-[2-(5-thiophen-2-yl-[1,3,4]oxadiazol-2-yl)-pyrrolidin-1-yl]-propionsäureethylester |
| 34 | (3-Dimethylamino-phenyl)-{2-[5-(3-methoxy-phenyl)-[1,3,4]oxadiazol-2-yl]-pyrrolidin-1-yl}-methanon |
| 35 | Essigsäure 2-oxo-2-[2-(5-phenyl-[1,3,4]oxadiazol-2-yl)-pyrrolidin-1-yl]-ethylester |
| 36 | (4-Ethoxy-phenyl)-[2-(5-pyridin-3-yl-[1,3,4]oxadiazol-2-yl)-pyrrolidin-1-yl]-methanon |
| 37 | 2-(2,5-Dimethoxy-phenyl)-1-[2-(5-pyridin-4-yl-[1,3,4]oxadiazol-2-yl)-pyrrolidin-1-yl]-ethanon |
| 38 | 3-Oxo-3-[2-(5-thiophen-2-yl-[1,3,4]oxadiazol-2-yl)-pyrrolidin-1-yl]-propionsäuremethylester |
| 39 | (2-Ethoxy-phenyl)-[2-(5-pyridin-4-yl-[1,3,4]oxadiazol-2-yl)-pyrrolidin-1-yl]-methanon |
| 40 | 3-[5-(1-Phenylmethansulfonyl-pyrrolidin-2-yl)-[1,3,4]oxadiazol-2-yl]-pyridin |
| 41 | 2-(5-Pyridin-4-yl-[1,3,4]oxadiazol-2-yl)-pyrrolidin-1-sulfonsäuredimethylamid |

(fortgesetzt)

| Beispiel - Nr. | Erfindungsgemäße Verbindung |
|---|---|
| | |
| 43 | {2-[5-(3-Methoxy-phenyl)-[1,3,4]oxadiazol-2-yl]-pyrrolidin-1-yl}-(4-trifluormethoxy-phenyl)-methanon |
| 44 | [2-(5-Pyridin-4-yl-[1,3,4]oxadiazol-2-yl)-pyrrolidin-1-yl]-thiophen-2-yl-methanon |
| 45 | [2-(5-Pyridin-4-yl-[1,3,4]oxadiazol-2-yl)-pyrrolidin-1-yl]-o-tolyl-methanon |
| 46 | 1-{2-[5-(4-Chlor-phenyl)-[1,3,4]oxadiazol-2-yl]-pyrrolidin-1-yl}-2-methoxy-ethanon |
| 47 | 5-{2-[5-(3-Methoxy-phenyl)-[1,3,4]oxadiazol-2-yl]-pyrrolidin-1-yl}-5-oxo-valeriansäuremethylester |
| 48 | 3-{2-[5-(3-Methoxy-phenyl)-[1,3,4]oxadiazol-2-yl]-pyrrolidin-1-yl}-3-oxo-propionsäuremethylester |
| 49 | Biphenyl-4-yl-[2-(5-phenyl-[1,3,4]oxadiazol-2-yl)-pyrrolidin-1-yl]-methanon |
| 50 | (2-Chlor-phenyl)-[2-(5-pyridin-4-yl-[1,3,4]oxadiazol-2-yl)-pyrrolidin-1-yl]-methanon |
| 51 | (2-Chlor-pyridin-3-yl)-[2-(5-thiophen-2-yl-[1,3,4]oxadiazol-2-yl)-pyrrolidin-1-yl]-methanon |
| 52 | 1-[2-(5-Thiophen-2-yl-[1,3,4]oxadiazol-2-yl)-pyrrolidin-1-yl]-butan-1-on |
| 53 | Essigsäure 1,1-dimethyl-2-oxo-2-[2-(5-phenyl-[1,3,4]oxadiazol-2-yl)-pyrrolidin-1-yl]-ethylester |
| 54 | [2-(5-Pyridin-4-yl-[1,3,4]oxadiazol-2-yl)-pyrrolidin-1-yl]-p-tolyl-methanon |
| 55 | (2,3-Dimethyl-phenyl)-[2-(5-pyridin-3-yl-[1,3,4]oxadiazol-2-yl)-pyrrolidin-1-yl]-methanon |
| 56 | 2-Cyclopentyl-1-[2-(5-pyridin-4-yl-[1,3,4]oxadiazol-2-yl)-pyrrolidin-1-yl]-ethanon |
| 57 | 3-{5-[1-(3-Chlor-benzylsulfonyl)-pyrrolidin-2-yl]-[1,3,4]oxadiazol-2-yl}-pyridin |
| 58 | 1-{2-[5-(3-Methoxy-phenyl)-[1,3,4]oxadiazol-2-yl]-pyrrolidin-1-yl}-pentan-1-on |
| | |
| 60 | Adamantan-1-yl-[2-(5-pyridin-3-yl-[1,3,4]oxadiazol-2-yl)-pyrrolidin-1-yl]-methanon |
| 61 | 2-(2-Fluor-phenyl)-5-[1-(2,4,6-trimethyl-benzylsulfonyl)-pyrrolidin-2-yl]-[1,3,4]oxadiazol |
| 62 | [2-(5-Pyridin-4-yl-[1,3,4]oxadiazol-2-yl)-pyrrolidin-1-yl]-(2-trifluormethyl-phenyl)-methanon |
| 63 | 2-[1-(3,4-Dimethoxy-benzylsulfonyl)-pyrrolidin-2-yl]-5-(2-fluor-phenyl)-[1,3,4]oxadiazol |
| 64 | Essigsäure 2-oxo-1-phenyl-2-[2-(5-pyridin-3-yl-[1,3,4]oxadiazol-2-yl)-pyrrolidin-1-yl]-ethylester |
| 65 | Furan-2-yl-{2-[5-(3-methoxy-phenyl)-[1,3,4]oxadiazol-2-yl]-pyrrolidin-1-yl}-methanon |
| 66 | (4-Brom-phenyl)-[2-(5-pyridin-3-yl-[1,3,4]oxadiazol-2-yl)-pyrrolidin-1-yl]-methanon |
| 67 | 2-(4-Chlor-phenoxy)-1-[2-(5-pyridin-4-yl-[1,3,4]oxadiazol-2-yl)-pyrrolidin-1-yl]-ethanon |
| 68 | 4-[5-(1-Benzylsulfonyl-pyrrolidin-2-yl)-[1,3,4]oxadiazol-2-yl]-pyridin |
| 69 | Furan-2-yl-[2-(5-thiophen-2-yl-[1,3,4]oxadiazol-2-yl)-pyrrolidin-1-yl]-methanon |
| 70 | 1-{2-[5-(2-Fluor-phenyl)-[1,3,4]oxadiazol-2-yl]-pyrrolidin-1-yl}-pent-4-en-1-on |
| 71 | 3-{5-[1-(2-Chlor-benzylsulfonyl)-pyrrolidin-2-yl]-[1,3,4]oxadiazol-2-yl}-pyridin |
| 72 | 4-{5-[1-(5-Chlor-thiophen-2-sulfonyl)-pyrrolidin-2-yl]-[1,3,4]oxadiazol-2-yl}-pyridin |
| 73 | [3-(2-Chlor-phenyl)-5-methyl-isoxazol-4-yl]-[2-(5-pyridin-3-yl-[1,3,4]oxadiazol-2-yl)-pyrrolidin-1-yl]-methanon |
| 74 | (5-Methyl-isoxazol-3-yl)-[2-(5-pyridin-3-yl-[1,3,4]oxadiazol-2-yl)-pyrrolidin-1-yl]-methanon |
| 75 | {2-[5-(2-Fluor-phenyl)-[1,3,4]oxadiazol-2-yl]-pyrrolidin-1-yl}-pentafluorphenyl-methanon |
| 76 | 4-Oxo-4-[2-(5-thiophen-2-yl-[1,3,4]oxadiazol-2-yl)-pyrrolidin-1-yl]-buttersäureethylester |

(fortgesetzt)

| Beispiel - Nr. | Erfindungsgemäße Verbindung |
|---|---|
| 77 | 2-(3-Methoxy-phenyl)-5-[1-(4-methoxy-2,3,6-trimethyl-benzylsulfonyl)-pyrrolidin-2-yl]-[1,3,4] oxadiazol |
| 78 | 4-{5-[1-(2,3,5,6-Tetramethyl-benzylsulfonyl)-pyrrolidin-2-yl]-[1,3,4]oxadiazol-2-yl}-pyridin |
| 79 | 1-[2-(5-Pyridin-4-yl-[1,3,4]oxadiazol-2-yl)-pyrrolidin-1-yl]-hexan-1-on |
| 80 | Cyclopentyl-[2-(5-pyridin-4-yl-[1,3,4]oxadiazol-2-yl)-pyrrolidin-1-yl]-methanon |
| 81 | (3-Chlor-2-fluor-phenyl)-{2-[5-(2-fluor-phenyl)-[1,3,4]oxadiazol-2-yl]-pyrrolidin-1-yl}-methanon |
| 82 | 2-[1-(4-Chlor-2,5-dimethyl-benzylsulfonyl)-pyrrolidin-2-yl]-5-(4-chlor-phenyl)-[1,3,4]oxadiazol |
| 83 | (4-Chlor-phenyl)-[2-(5-pyridin-3-yl-[1,3,4]oxadiazol-2-yl)-pyrrolidin-1-yl]-methanon |
| 84 | 3-Phenyl-1-[2-(5-phenyl-[1,3,4]oxadiazol-2-yl)-pyrrolidin-1-yl]-propenon |
| 85 | (5-Methyl-2-phenyl-2H-[1,2,3]triazol-4-yl)-[2-(5-pyridin-4-yl-[1,3,4]oxadiazol-2-yl)-Pyrrolidin-1-yl]-methanon |
| 86 | 3-{5-[1-(2,4-Difluor-benzylsulfonyl)-pyrrolidin-2-yl]-[1,3,4]oxadiazol-2-yl}-pyridin |
| 87 | 2-Benzyloxy-1-{2-[5-(3-methoxy-phenyl)-[1,3,4]oxadiazol-2-yl]-pyrrolidin-1-yl}-ethanon |
| 88 | (6-Chlor-pyridin-3-yl)-[2-(5-thiophen-2-yl-[1,3,4]oxadiazol-2-yl)-pyrrolidin-1-yl]-methanon |
| 89 | 3,3-Dimethyl-1-[2-(5-thiophen-2-yl-[1,3,4]oxadiazol-2-yl)-pyrrolidin-1-yl]-butan-1-on |
| 90 | (2-Ethoxy-phenyl)-{2-[5-(3-methoxy-phenyl)-[1,3,4]oxadiazol-2-yl]-pyrrolidin-1-yl}-methanon |
| 91. | (4-Ethyl-phenyl)-[2-(5-pyridin-4-yl-[1,3,4]oxadiazol-2-yl)-pyrrolidin-1-yl]-methanon |
| 92 | (4-Brom-phenyl)-{2-[5-(3-methoxy-phenyl)-[1,3,4]oxadiazol-2-yl]-pyrrolidin-1-yl}-methanon |
| 93 | (5-Methyl-isoxazol-3-yl)-[2-(5-phenyl-[1,3,4]oxadiazol-2-yl)-pyrrolidin-1-yl]-methanon |
| 94 | (2-Chlor-pyridin-4-yl)-{2-[5-(3-methoxy-phenyl)-[1,3,4]oxadiazol-2-yl]-pyrrolidin-1-yl}-methanon |
| 95 | {2-[5-(2-Fluor-phenyl)-[1,3,4]oxadiazol-2-yl]-pyrrolidin-1-yl}-(4-nitro-phenyl)-methanon |
| 96 | 1-{2-[5-(3-Methoxy-phenyl)-[1,3,4]oxadiazol-2-yl]-pyrrolidin-1-yl}-3-phenyl-propan-1-on |
| 97 | (3-Chlor-thiophen-2-yl)-{2-[5-(3-methoxy-phenyl)-[1,3,4]oxadiazol-2-yl]-pyrrolidin-1-yl}-methanon |
| 98 | 2-(2-Fluor-phenyl)-5-[1-(toluene-4-sulfonyl)-pyrrolidin-2-yl]-[1,3,4]oxadiazol |
| 99 | 4-{5-[1-(4-Butoxy-benzylsulfonyl)-pyrrolidin-2-yl]-[1,3,4]oxadiazol-2-yl}-pyridin |
| 100 | Cyclopropyl-[2-(5-phenyl-[1,3,4]oxadiazol-2-yl)-pyrrolidin-1-yl]-methanon |
| 101 | (2,6-Difluor-phenyl)-[2-(5-pyridin-4-yl-[1,3,4]oxadiazol-2-yl)-pyrrolidin-1-yl]-methanon |
| 102 | (2,5-Dimethyl-furan-3-yl)-[2-(5-pyridin-4-yl-[1,3,4]oxadiazol-2-yl)-pyrrolidin-1-yl]-methanon |
| 103 | 2-[2-(5-Pyridin-3-yl-[1,3,4]oxadiazol-2-yl)-pyrrolidin-1-sulfonyl]-benzoesäuremethylester |
| 104 | 2-Ethyl-1-[2-(5-thiophen-2-yl-[1,3,4]oxadiazol-2-yl)-pyrrolidin-1-yl]-butan-1-on |
| 105 | (3-Difluormethylsulfanyl-phenyl)-[2-(5-pyridin-4-yl-[1,3,4]oxadiazol-2-yl)-pyrrolidin-1-yl]-methan |
| 106 | Benzo[1,2,5]oxadiazol-5-yl-{2-[5-(3-methoxy-phenyl)-[1,3,4]oxadiazol-2-yl]-pyrrolidin-1-yl}-methanon |
| 107 | 3-{5-[1-(Toluol-4-sulfonyl)-pyrrolidin-2-yl]-[1,3,4]oxadiazol-2-yl}-pyridin |
| 108 | 4-{5-[1-(3-Trifluormethyl-benzylsulfonyl)-pyrrolidin-2-yl]-[1,3,4]oxadiazol-2-yl}-pyridin |
| 109 | (4-Ethoxy-phenyl)-[2-(5-phenyl-[1,3,4]oxadiazol-2-yl)-pyrrolidin-1-yl]-methanon |
| 110 | 2-(2-Brom-phenyl)-1-[2-(5-pyridin-4-yl-[1,3,4]oxadiazol-2-yl)-pyrrolidin-1-yl]-ethanon |
| 111 | [2-(5-Pyridin-4-yl-[1,3,4]oxadiazol-2-yl)-pyrrolidin-1-yl]-(4-trifluormethyl-phenyl)-methanon |

(fortgesetzt)

| Beispiel - Nr. | Erfindungsgemäße Verbindung |
|---|---|
| 112 | 2-Thiophen-2-yl-5-[1-(2,4,6-trimethyl-benzylsulfonyl)-pyrrolidin-2-yl]-[1,3,4]oxadiazol |
| 113 | 1-{4-[2-(5-Phenyl-[1,3,4]oxadiazol-2-yl)-pyrrolidin-1-sulfonyl]-phenyl}- ethanon |
| 114 | Furan-2-yl-[2-(5-phenyl-[1,3,4]oxadiazol-2-yl)pyrrolidin-1-yl]-methanon |
| 115 | (3,5-Difluor-phenyl)-[2-(5-pyridin-4-yl-[1,3,4]oxadiazol-2-yl)-pyrrolidin-1-yl]-methanon |
| 116 | [2-(5-Biphenyl-4-yl-[1,3,4]oxadiazol-2-yl)-pyrrolidin-1-yl]-(4-nitro-phenyl)-methanon |
| 117 | (4-Fluor-phenyl)-{2-[5-(2-fluor-phenyl)-[1,3,4]oxadiazol-2-yl]-pyrrolidin-1-yl}-methanon |
| 118 | {2-[5-(3-Methoxy-phenyl)-[1,3,4]oxadiazol-2-yl]-pyrrolidin-1-yl}-(4-propyl-phenyl)-methanon |
| 119 | (3,4-Difluor-phenyl)-[2-(5-pyridin-4-yl-[1,3,4]oxadiazol-2-yl)-pyrrolidin-1-yl]-methanon |
| 120 | (5-Fluor-2-trifluomethyl-phenyl)-[2-(5-pyridin-4-yl-[1,3,4]oxadiazol-2-yl)-pyrrolidin-1-yl]-methanon |
| 121 | 2-Phenyl-1-[2-(5-phenyl-[1,3,4]oxadiazol-2-yl)-pyrrolidin-1-yl]-ethanon |
| 122 | [2-(5-Pyridin-3-yl-[1,3,4]oxadiazol-2-yl)-pyrrolidin-1-yl]-(3-trifluormethoxy-phenyl)-methanon |
| 123 | (4-Fluor-phenyl)-{2-[5-(3-methoxy-phenyl)-[1,3,4]oxadiazol-2-yl]-pyrrolidin-1-yl}-methanon |
| 124 | (4-Propyl-phenyl)-[2-(5-pyridin-4-yl-[1,3,4]oxadiazol-2-yl)-pyrrolidin-1-yl]-methanon |
| 125 | 2-Phenyl-1-[2-(5-phenyl-[1,3,4]oxadiazol-2-yl)-pyrrolidin-1-yl]-butan-1-on |
| 126 | [2-(5-Phenyl-[1,3,4]oxadiazol-2-yl)-pyrrolidin-1-yl]-(4-trifluormethoxy-phenyl)-methanon |
| 127 | (3-Nitro-phenyl)-[2-(5-pyridin-4-yl-[1,3,4]oxadiazol-2-yl)-pyrrolidin-1-yl]-methanon |
| 128 | 2-Ethyl-1-{2-[5-(2-fluor-phenyl)-[1,3,4]oxadiazol-2-yl]-pyrrolidin-1-yl}-butan-1-on |
| 129 | (5-Fluor-2-trifluormethyl-phenyl)-[2-(5-phenyl-[1,3,4]oxadiazol-2-yl)-pyrrolidin-1-yl]-methanon |
| 130 | 1-{2-[5-(2-Fluor-phenyl)-[1,3,4]oxadiazol-2-yl]-pyrrolidin-1-yl}-2-propyl-pentan-1-on |
| 131 | {2-[5-(3-Methoxy-phenyl)-[1,3,4]oxadiazol-2-yl]-pyrrolidin-1-yl}-(5-methyl-2-phenyl-2H-[1,2,3] triazol-4-yl)-methanon |
| 132 | [2-(5-Phenyl-[1,3,4]oxadiazol-2-yl)-pyrrolidin-1-yl]-(2-trifluormethyl-phenyl)-methanon |
| 133 | 2-(3-Methoxy-phenyl)-1-[2-(5-phenyl-[1,3,4]oxadiazol-2-yl)-pyrrolidin-1-yl]-ethanon |
| 134 | 3-{5-[1-(4-Trifluormethoxy-benzylsulfonyl)-pyrrolidin-2-yl]-[1,3,4]oxadiazol-2-yl}-pyridin |
| 135 | {2-[5-(4-Chlor-phenyl)-[1,3,4]oxadiazol-2-yl]-pyrrolidin-1-yl}-furan-2-yl-methanon |
| 136 | {2-[5-(2-Fluor-phenyl)-[1,3,4]oxadiazol-2-yl]-pyrrolidin-1-yl}-furan-2-yl-methanon |
| 137 | (4-Methyl-3-nitro-phenyl)-[2-(5-pyridin-4-yl-[1,3,4]oxadiazol-2-yl)-pyrrolidin-1-yl]-methanon |
| 138 | (4-Methoxy-phenyl)-[2-(5-phenyl-[1,3,4]oxadiazol-2-yl)-pyrrolidin-1-yl]-methanon |
| 139 | (2-Chlor-pyridin-4-yl)-{2-[5-(2-fluor-phenyl)-[1,3,4]oxadiazol-2-yl]-pyrrolidin-1-yl}-methanon |
| 140 | (3-Brom-phenyl)-[2-(5-phenyl-[1,3,4]oxadiazol-2-yl)-pyrrolidin-1-yl]-methanon |
| 141 | 2-Propyl-1-[2-(5-pyridin-3-yl-[1,3,4]oxadiazol-2-yl)-pyrrolidin-1-yl]-pentan-1-on |
| 142 | 2-(4-Chlor-phenyl)-5-{1-[4-(1,1-dimethyl-propyl)-benzylsulfonyl]-pyrrolidin-2-yl}-[1,3,4]oxadiazol |
| 143 | (3-Chlor-phenyl)-[2-(5-pyridin-3-yl-[1,3,4]oxadiazol-2-yl)-pyrrolidin-1-yl]-methanon |
| 144 | (3-Chlor-4-fluor-phenyl)-[2-(5-pyridin-3-yl-[1,3,4]oxadiazol-2-yl)-pyrrolidin-1-yl]-methanon |
| 145 | (2,6-Difluor-3-methyl-phenyl)-[2-(5-pyridin-4-yl-[1,3,4]oxadiazol-2-yl)-pyrrolidin-1-yl]-methanon |
| 146 | (3-Fluor-4-methyl-phenyl)-{2-[5-(3-methoxy-phenyl)-[1,3,4]oxadiazol-2-yl]-pyrrolidin-1-yl}-methanon |

(fortgesetzt)

| Beispiel - Nr. | Erfindungsgemäße Verbindung |
|---|---|
| 147 | {2-[5-(2-Fluor-phenyl)-[1,3,4]oxadiazol-2-yl]-pyrrolidin-1-yl}-(4-methyl-[1,2,3]thiadiazol-5-yl)-methanon |
| 148 | 2-Thiophen-2-yl-5-[1-(toluol-4-sulfonyl)-pyrrolidin-2-yl]-[1,3,4]oxadiazol |
| 149 | {2-[5-(4-Chlor-phenyl)-[1,3,4]oxadiazol-2-yl]-pyrrolidin-1-yl}-(3,4-dimethoxy-phenyl)-methanon |
| 150 | {2-[5-(4-Chlor-phenyl)-[1,3,4]oxadiazol-2-yl]-pyrrolidin-1-yl}-(4-ethyl-phenyl)-methanon |
| 151 | (4-tert-Butyl-phenyl)-[2-(5-phenyl-[1,3,4]oxadiazol-2-yl)-pyrrolidin-1-yl]-methanon |
| 152 | Naphth-1-yl-[2-(5-phenyl-[1,3,4]oxadiazol-2-yl)-pyrrolidin-1-yl]-methanon |
| 153 | 1-[2-(5-Phenyl-[1,3,4]oxadiazol-2-yl)-pyrrolidin-1-yl]-pent-4-en-1-on |
| 154 | {2-[5-(2-Fluor-phenyl)-[1,3,4]oxadiazol-2-yl]-pyrrolidin-1-yl}-(4-propyl-phenyl)-methanon |
| 155 | 1-{2-[5-(2-Fluor-phenyl)-[1,3,4]oxadiazol-2-yl]-pyrrolidin-1-yl}-3-phenyl-propenon |
| 156 | 1-{2-[5-(4-Chlor-phenyl)-[1,3,4]oxadiazol-2-yl]-pyrrolidin-1-yl}-3,3-dimethyl-butan-1-on |
| 157 | 2-[1-(4-Chlor-benzylsulfonyl)-pyrrolidin-2-yl]-5-(4-chlor-phenyl)-[1,3,4]oxadiazol |
| 158 | Adamantan-1-yl-[2-(5-phenyl-[1,3,4]oxadiazol-2-yl)-pyrrolidin-1-yl]-methanon |
| 159 | {2-[5-(2-Fluor-phenyl)-[1,3,4]oxadiazol-2-yl]-pyrrolidin-1-yl}-o-tolyl-methanon |
| 160 | Benzo[1,2,5]oxadiazol-5-yl-[2-(5-phenyl-[1,3,4]oxadiazol-2-yl)-pyrrolidin-1-yl]-methanon |
| 161 | (4-Nitro-phenyl)-[2-(5-phenyl-[1,3,4]oxadiazol-2-yl)-pyrrolidin-1-yl]-methanon |
| 162 | 3-(2-Chlor-phenyl)-1-{2-[5-(2-fluor-phenyl)-[1,3,4]oxadiazol-2-yl]-pyrrolidin-1-yl}-propenon |
| 163 | 4-{5-[1-(4-Ethyl-benzylsulfonyl)-pyrrolidin-2-yl]-[1,3,4]oxadiazol-2-yl}-pyridin |
| 164 | (2,5-Bis-trifluormethyl-phenyl)-{2-[5-(4-chlor-phenyl)-[1,3,4]oxadiazol-2-yl]-pyrrolidin-1-yl}-methanon |
| 165 | (2,5-Bis-trifluormethyl-phenyl)-[2-(5-thiophen-2-yl-[1,3,4]oxadiazol-2-yl)-pyrrolidin-1-yl]-methanon |
| 166 | (1-Phenyl-5-propyl-1H-pyrazol-4-yl)-[2-(5-pyridin-3-yl-[1,3,4]oxadiazol-2-yl)-pyrrolidin-1-yl]-methanon |
| 167 | 1-[2-(5-Pyridin-4-yl-[1,3,4]oxadiazol-2-yl)-pyrrolidin-1-yl]-3-(3-trifluormethyl-phenyl)-propenon |
| 168 | (2-Methylsulfanyl-pyridin-3-yl)-[2-(5-thiophen-2-yl-[1,3,4]oxadiazol-2-yl)-pyrrolidin-1-yl]-methanon |
| 169 | (2,5-Dimethyl-furan-3-yl)-{2-[5-(3-methoxy-phenyl)-[1,3,4]oxadiazol-2-yl]-pyrrolidin-1-yl}-methanon |
| 170 | {2-[5-(4-Chlor-phenyl)-[1,3,4]oxadiazol-2-yl]-pyrrolidin-1-yl}-(5-fluor-2-trifluormethyl-phenyl)-methanon |
| 171 | (3-Chlor-phenyl)-[2-(5-thiophen-2-yl-[1,3,4]oxadiazol-2-yl)-pyrrolidin-1-yl]-methanon |
| 172 | (4-Chlor-phenyl)-[2-(5-thiophen-2-yl-[1,3,4]oxadiazol-2-yl)-pyrrolidin-1-yl]-methanon |
| 173 | (2,3-Difluor-phenyl)-{2-[5-(3-methoxy-phenyl)-[1,3,4]oxadiazol-2-yl]-pyrrolidin-1-yl}-methanon |
| 174 | (2-Methyl-6-trifluormethyl-pyridin-3-yl)-[2-(5-phenyl-[1,3,4]oxadiazol-2-yl)-pyrrolidin-1-yl]-methanon |
| 175 | 1-{2-[5-(4-Chlor-phenyl)-[1,3,4]oxadiazol-2-yl]-pyrrolidin-1-yl}-2-ethyl-butan-1-on |
| 176 | 1-{2-[5-(4-Chlor-phenyl)-[1,3,4]oxadiazol-2-yl]-pyrrolidin-1-yl}-pent-4-en-1-on |
| 177 | [2-(5-Biphenyl-4-yl-[1,3,4]oxadiazol-2-yl)-pyrrolidin-1-yl]-pentafluorphenyl-methanon |
| 178 | (3-Fluor-4-trifluormethyl-phenyl)-[2-(5-thiophen-2-yl-[1,3,4]oxadiazol-2-yl)-pyrrolidin-1-yl]-methanon |
| 179 | 1-{2-[5-(2-Fluor-phenyl)-[1,3,4]oxadiazol-2-yl]-pyrrolidin-1-yl}-4-phenoxy-butan-1-on |

(fortgesetzt)

| Beispiel - Nr. | Erfindungsgemäße Verbindung |
|---|---|
| 180 | [1-(4-Chlor-phenyl)-5-trifluormethyl-1H-pyrazol-4-yl]-{2-[5-(2-fluor-phenyl)-[1,3,4]oxadiazol-2-yl]-pyrrolidin-1-yl}-methanon |
| 18.1 | [2-(5-Phenyl-[1,3,4]oxadiazol-2-yl)-pyrrolidin-1-yl]-(4-trifluormethylsulfanyl-phenyl)-methanon |
| 182 | 2,2,2-Trifluor-1-{7-[2-(5-pyridin-4-yl-[1,3,4]oxadiazol-2-yl)-pyrrolidin-1-sulfonyl]-3,4-dihydro-1H-isochinolin-2-yl}-ethanon |
| 183 | 1-[2-(5-Biphenyl-4-yl-[1,3,4]oxadiazol-2-yl)-pyrrolidin-1-yl]-2-methoxy-ethanon |
| 184 | (2-Chlor-5-trifluormethyl-phenyl)-[2-(5-pyridin-4-yl-[1,3,4]oxadiazol-2-yl)-pyrrolidin-1-yl]-methanon |
| 185 | (2-Chlor-5-trifluormethyl-phenyl)-[2-(5-phenyl-[1,3,4]oxadiazol-2-yl)-pyrrolidin-1-yl]-methanon |
| 186 | 1-{2-[5-(4-Chlor-phenyl)-[1,3,4]oxadiazol-2-yl]-pyrrolidin-1-yl}-2-phenoxy-ethanon |
| 187 | (2,3-Difluor-4-methyl-phenyl)-{2-[5-(3-methoxy-phenyl)-[1,3,4]oxadiazol-2-yl]-pyrrolidin-1-yl}-methanon |
| 188 | 2-[2-(5-Phenyl-[1,3,4]oxadiazol-2-yl)-pyrrolidin-1-sulfonyl]-benzoesäuremethylester |
| 189 | 2-Biphenyl-4-yl-5-{1-[4-(1,1-dimethyl-propyl)-benzylsulfonyl]-pyrrolidin-2-yl}-[1,3,4]oxadiazol |
| 190 | 3-Cyclopentyl-1-[2-(5-phenyl-[1,3,4]oxadiazol-2-yl)-pyrrolidin-1-yl]-propan-1-on |
| 191 | 3,3-Dimethyl-1-[2-(5-phenyl-[1,3,4]oxadiazol-2-yl)-pyrrolidin-1-yl]-butan-1-on |
| 192 | (2-Chlor-4-nitro-phenyl)-[2-(5-phenyl-[1,3,4]oxadiazol-2-yl)-pyrrolidin-1-yl]-methanon |
| 193 | 1-[2-(5-Biphenyl-4-yl-[1,3,4]oxadiazol-2-yl)-pyrrolidin-1-yl]-2-(2-methoxy-ethoxy)-ethanon |
| 194 | 1-{2-[5-(4-Chlor-phenyl)-[1,3,4]oxadiazol-2-yl]-pyrrolidin-1-yl}-2-ethyl-hexan-1-on |
| 195 | 1-[2-(5-Biphenyl-4-yl-[1,3,4]oxadiazol-2-yl)-pyrrolidin-1-yl]-2-phenyl-butan-1-on |
| 196 | (2,3-Dimethyl-phenyl)-{2-[5-(2-fluor-phenyl)-[1,3,4]oxadiazol-2-yl]-pyrrolidin-1-yl}-methanon |
| 197 | (2,5-Bis-trifluormethyl-phenyl)-[2-(5-pyridin-4-yl-[1,3,4]oxadiazol-2-yl)-pyrrolidin-1-yl]-methanon |
| 198 | 2-(4-Chlor-phenoxy)-1-[2-(5-phenyl-[1,3,4]oxadiazol-2-yl)-pyrrolidin-1-yl]-ethanon |
| 199 | (2-Ethoxy-phenyl)-[2-(5-phenyl-[1,3,4]oxadiazol-2-yl)-pyrrolidin-1-yl]-methanon |
| 200 | (5-tert-Butyl-2-methyl-2H-pyrazol-3-yl)-[2-(5-phenyl-[1,3,4]oxadiazol-2-yl)-pyrrolidin-1-yl]-methanon |
| 201 | (4-Methyl-[1,2,3]thiadiazol-5-yl)-[2-(5-thiophen-2-yl-[1,3,4]oxadiazol-2-yl)-pyrrolidin-1-yl]-methanon |
| 202 | [2-(5-Biphenyl-4-yl-[1,3,4]oxadiazol-2-yl)-pyrrolidin-1-yl]-(3,4-dichlor-phenyl)-methanon |
| 203 | (4-Propyl-phenyl)-[2-(5-thiophen-2-yl-[1,3,4]oxadiazol-2-yl)-pyrrolidin-1-yl]-methanon |
| 204 | (3,4-Difluor-phenyl)-[2-(5-phenyl-[1,3,4]oxadiazol-2-yl)-pyrrolidin-1-yl]-methanon |
| 205 | (3-Chlor-2-fluor-phenyl)-[2-(5-thiophen-2-yl-[1,3,4]oxadiazol-2-yl)-pyrrolidin-1-yl]-methanon |
| 206 | (2-Chlor-phenyl)-[2-(5-phenyl-[1,3,4]oxadiazol-2-yl)-pyrrolidin-1-yl]-methanon |
| 207 | [2-(5-Biphenyl-4-yl-[1,3,4]oxadiazol-2-yl)-pyrrolidin-1-yl]-m-tolyl-methanon |
| 208 | 1-{2-[5-(4-Chlor-phenyl)-[1,3,4]oxadiazol-2-yl]-pyrrolidin-1-yl}-butan-1-on |
| 209 | {2-[5-(4-Chlor-phenyl)-[1,3,4]oxadiazol-2-yl]-pyrrolidin-1-yl}-pyridin-2-yl-methanon |
| 210 | [2-(5-Phenyl-[1,3,4]oxadiazol-2-yl)-pyrrolidin-1-yl]-(4-trifluormethyl-phenyl)-methanon |
| 211 | 2-Biphenyl-4-yl-5-[1-(4-chlor-benzylsulfonyl)-pyrrolidin-2-yl]-[1,3,4]oxadiazol |
| 212 | 1-[2-(5-Biphenyl-4-yl-[1,3,4]oxadiazol-2-yl)-pyrrolidin-1-yl]-2-phenoxy-ethanon |
| 213 | 2-Phenyl-5-[1-(toluol-4-sulfonyl)-pyrrolidin-2-yl]-[1,3,4]oxadiazol |

(fortgesetzt)

| Beispiel - Nr. | Erfindungsgemäße Verbindung |
|---|---|
| 214 | [2-(5-Phenyl-[1,3,4]oxadiazol-2-yl)-pyrrolidin-1-yl]-(3-trifluormethoxy-phenyl}-methanon |
| 215 | [2-(5-Biphenyl-4-yl-[1,3,4]oxadiazol-2-yl)-pyrrolidin-1-yl]-(4-propyl-phenyl)-methanon |
| 216 | Benzoesäure 2-[2-(5-phenyl-[1,3,4]oxadiazol-2-yl)-pyrrolidin-1-carbonyl]-benzylester |
| 217 | {2-[5-(4-Chlor-phenyl)-[1,3,4]oxadiazol-2-yl]-pyrrolidin-1-yl}-(4-nitro-phenyl)-methanon |
| 218 | 2-Phenoxy-1-[2-(5-thiophen-2-yl-[1,3,4]oxadiazol-2-yl)-pyrrolidin-1-yl]-propan-1-on |
| 219 | 2-(4-Chlor-phenyl)-1-[2-(5-phenyl-[1,3,4]oxadiazol-2-yl)-pyrrolidin-1-yl]-ethanon |
| 220 | [2-(5-Biphenyl-4-yl-[1,3,4]oxadiazol-2-yl)-pyrrolidin-1-yl]-(2-chlor-5-trifluormethyl-phenyl)-methanon |
| 221 | (2,3-Difluor-phenyl)-[2-(5-phenyl-[1,3,4]oxadiazol-2-yl)-pyrrolidin-1-yl]-methanon |
| 222 | 2-Biphenyl-4-yl-5-[1-(3-chlor-4-fluor-benzylsulfonyl)-pyrrolidin-2-yl]-[1,3,4]oxadiazol |
| 223 | (3,4-Difluor-phenyl)-[2-(5-thiophen-2-yl-[1,3,4]oxadiazol-2-yl)-pyrrolidin-1-yl]-methanon |
| 224 | 1-[2-(5-Biphenyl-4-yl-[1,3,4]oxadiazol-2-yl)-pyrrolidin-1-yl]-2-propyl-pentan-1-on |
| 225 | (3-Fluor-4-trifluormethyl-phenyl)-[2-(5-pyridin-4-yl-[1,3,4]oxadiazol-2-yl)-pyrrolidin-1-yl]-methanon |
| 226 | (6-Chlor-2-fluor-3-methyl-phenyl)-{2-[5-(4-chlor-phenyl)-[1,3,4]oxadiazol-2-yl]-pyrrolidin-1-yl}-methanon |
| 227 | (2-Phenyl-cyclopropyl)-[2-(5-phenyl-[1,3,4]oxadiazol-2-yl)-pyrrolidin-1-yl]-methanon |
| 228 | (4-Brom-3-methyl-phenyl)-[2-(5-phenyl-[1,3,4]oxadiazol-2-yl)-pyrrolidin-1-yl]-methanon |
| 229 | [2-(5-Biphenyl-4-yl-[1,3,4]oxadiazol-2-yl)-pyrrolidin-1-yl]-(2,3-dimethyl-phenyl)-methanon |
| 230 | [2-(5-Biphenyl-4-yl-[1,3,4]oxadiazol-2-yl)-pyrrolidin-1-yl]-(3-chlor-phenyl)-methanon |
| 231 | [2-(5-Biphenyl-4-yl-[1,3,4]oxadiazol-2-yl)-pyrrolidin-1-yl]-(2,3-difluor-4-methyl-phenyl)-methanon |
| 232 | (2-Chlor-6-fluor-phenyl)-{2-[5-(4-chlor-phenyl)-[1,3,4]oxadiazol-2-yl]-pyrrolidin-1-yl}-methanon |
| 233 | [2-(5-Biphenyl-4-yl-[1,3,4]oxadiazol-2-yl)-pyrrolidin-1-yl]-(5-tert-butyl-2-methyl-2H-pyrazol-3-yl)-methanon |
| 234 | {2-[5-(4-Chlor-phenyl)-[1,3,4]oxadiazol-2-yl]-pyrrolidin-1-yl}-(2,3-dichlor-phenyl)-methanon |
| 235 | 2-(5-Pyridin-3-yl-[1,3,4]oxadiazol-2-yl)-pyrrolidin-1-carbonsäure (4-ethoxy-phenyl)-amid |
| 236 | 2-(5-Pyridin-3-yl-[1,3,4]oxadiazol-2-yl)-pyrrolidin-1-carbonsäure (2,5-dichlor-phenyl)-amid |
| 237 | 2-[5-(3-Methoxy-phenyl)-[1,3,4]oxadiazol-2-yl]-pyrrolidin-1-carbonsäure 4-fluor-benzylamid |
| 238 | 2-[5-(2-Fluor-phenyl)-[1,3,4]oxadiazol-2-yl]-pyrrolidin-1-carbonsäure (2,5-dimethoxy-phenyl)-amid |
| 239 | 2-(5-Biphenyl-4-yl-[1,3,4]oxadiazol-2-yl)-pyrrolidin-1-carbonsäure (2,5-dimethoxy-phenyl)-amid |
| 240 | 2-(5-Biphenyl-4-yl-[1,3,4]oxadiazol-2-yl)-pyrrolidin-1-carbonsäure (2,5-difluor-phenyl)-amid |
| 241 | 2-(5-Biphenyl-4-yl-[1,3,4]oxadiazol-2-yl)-pyrrolidin-1-carbonsäure (3-cyano-phenyl)-amid |
| 242 | 2-(5-Biphenyl-4-yl-[1,3,4]oxadiazol-2-yl)-pyrrolidin-1-carbonsäure (3-cyano-phenyl)-amid |
| 243 | 2-[5-(3-Methoxy-phenyl)-[1,3,4]oxadiazol-2-yl]-pyrrolidin-1-carbonsäure (3-cyano-phenyl)-amid |
| 244 | 2-(5-Phenyl-[1,3,4]oxadiazol-2-yl)-pyrrolidin-1-carbonsäure (4-chlor-3-trifluormethyl-phenyl)-amid |
| 245 | 2-(5-Phenyl-[1,3,4]oxadiazol-2-yl)-pyrrolidin-1-carbonsäure (4-methoxy-phenyl)-amid |
| 246 | 2-(5-Biphenyl-4-yl-[1,3,4]oxadiazol-2-yl)-pyrrolidin-1-carbonsäure (4-trifluormethoxy-phenyl)-amid |
| 247 | 1-{2-[5-(3-Chlor-phenyl)-[1,3,4]oxadiazol-2-yl]-pyrrolidin-1-yl}-3-cyclopentyl-propan-1-on |
| 248 | 1-{2-[5-(3-Chlor-phenyl)-[1,3,4]oxadiazol-2-yl]-pyrrolidin-1-yl}-pentan-1-on |

Die Herstellung der erfindungsgemäßen Verbindungen erfolgte gemäß oben beschriebenen Verfahren und wird im einzelnen anhand der folgenden beiden Beispiele aufgezeigt:

**Beispiel 247:**

1-{2-[5-(3-Chlor-phenyl)-[1,3,4]oxadiazol-2-yl]-pyrrolidin-1-yl}-3-cyclopentyl-propan-1-on

1. Stufe

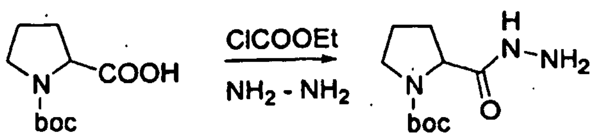


**a** **b**

Eine Lösung von Ethylchlorformiat (10,33 ml) in 20 ml trockenem THF wurde innerhalb von 5 min zu einer gerührten und auf -15°C gekühlten Lösung von Boc-(L)-Prolin (23,27 g) und 15,07 ml Triethylamin in 250 ml trockenem THF zugetropft. Es wurde weitere 30 min bei -15°C gerührt. Die kalte Reaktionsmischung wurde abfiltriert, das Filtrat auf ein Volumen von etwa 125 ml eingeengt und dann innerhalb von 15 min zu einer gerührten und gekühlten (0°C) Lösung von Hydrazinmonohydrat (10,5 ml) in 150 ml trockenem THF zugetropft. Das Reaktionsgemisch wurde weitere 15 min bei 0°C und anschließend weitere 2 h bei Raumtemperatur gerührt. Die Lösung wurde von einem milchig-weiße Öl am Boden des Kolbens abdekantiert und zur Trockne eingeengt. Das Rohprodukt wurde ohne weitere Aufreinigung eingesetzt. Ausbeute: 25,63 g.

2. Stufe:

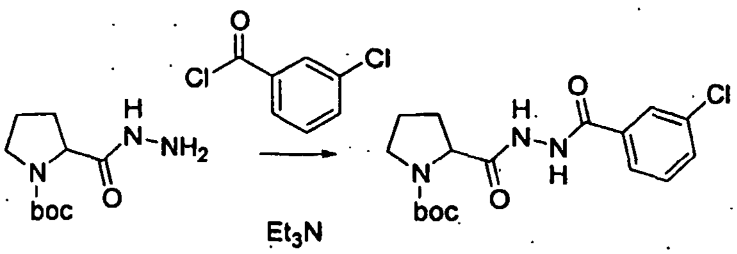


**b** **c**

11,42 ml 3-Chlorbenzoylchlorid in 10 ml trockenem THF wurde innerhalb von 5 min zu einer gerührten und auf -15°C gekühlten Lösung des Hydrazides b (18,6 g) und Triethylamin (13 ml) in 180 ml trockenem THF getropft. Weitere 20 min wurde bei -15°C gerührt, 60 min bei 0°C und dann 2 h bei Raumtemperatur. Das Reaktionsgemisch wurde abfiltriert und das Filtrat wurde zur Trockne eingeengt. Das braune, ölige Rohprodukt wurde durch Kieselgelchromatographie (DCM, 3.75% Methanol). Ausbeute: 18,67 g.

3. Stufe

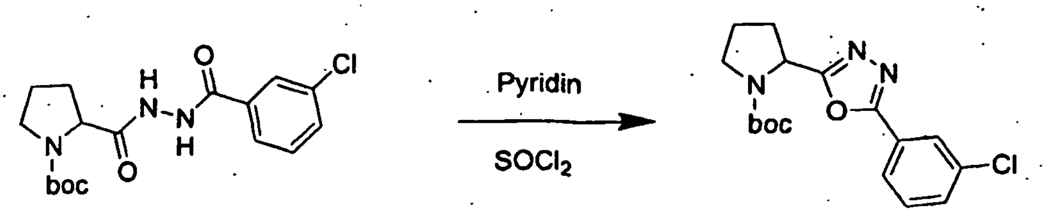


c d

Der Ringschluss wurde in wasserfreiem Diethylether unter Stickstoffatmosphäre durchgeführt. 6,3 g $SOCl_2$ wurde zu einer auf 0°C gekühlten Lösung des Hydrazids c (15 g) und 8,4 g Pyridin in trockenem Diethylether getropft. Das Reaktionsgemisch wurde 2h bei 0°C gerührt. Das entstehende Salz wurde abfiltriert und das Filtrat bei 0°C eingeengt. Der Rückstand wurde in 500 ml Toluol gelöst und unter Stickstoffatmosphäre zum Rückfluss erwärmt. Nach 2 h wurde die Lösung zur Trockne eingeengt und über Kieselgelchromatographie (Diethylether) gereinigt. Ausbeute: 13 g.

4. Stufe

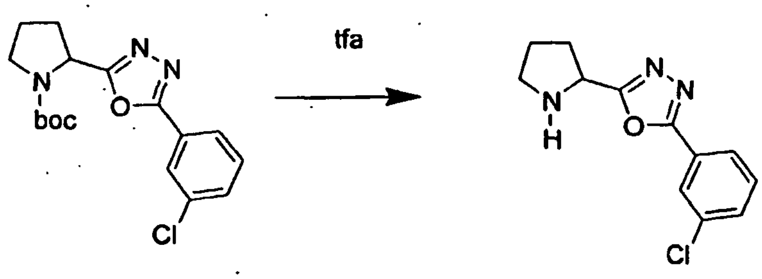


d e

13 g des Oxadiazol-Derivates d wurden in 25 ml DCM gelöst. Nach Zugabe von 5 ml Trifluoressigsäure (Tfa) wurde 6 Tage lang unter Stickstoffatmosphäre gerührt. Nach einer weiteren Zugabe von 10 ml Trifluoressigsäure wurde weitere 6 h gerührt und das Reaktionsgemisch daraufhin zur Trockne eingeengt. 100 ml einer gesättigten, wässrigen $NaHCO_3$-Lösung und 100 ml DCM wurden zugegeben und die wässrige Phase zwei weitere Male mit DCM extrahiert. Die vereinigten organischen Phasen wurden über $Na_2SO_4$ getrocknet und zur Trockne eingeengt. Das Produkt wurde ohne weitere Aufreinigung eingesetzt. Ausbeute: 10 g.

5. Stufe

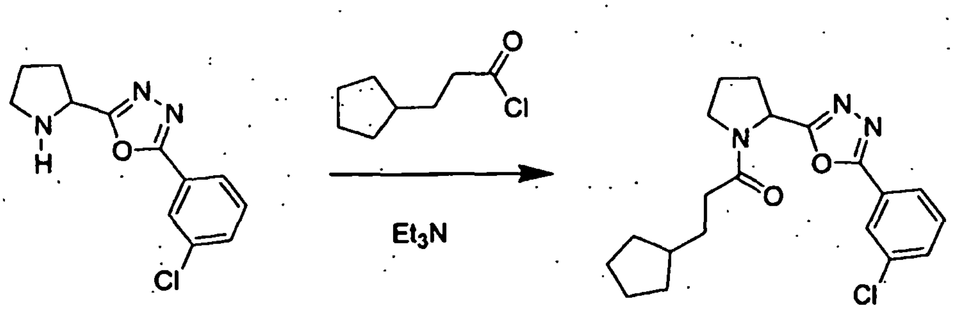


e f

804 mg des Oxadiazol-Derivates e wurden in 9 ml DCM gelöst. Bei einer Temperatur von - 20°C wurden 326 mg Triethylamin und 517 mg 3-Cyclopentylpropionylchlorid in 1 ml DCM zugegeben. Das Reaktionsgemisch wurde 2 h bei - 20°C gerührt und anschließend zur Trockne eingeengt. Das Produkt wurde durch Säulenchromatographie gereinigt (Kieselgel, Ethylacetat/Heptan 1:2, danach basisches Allox, Ethylacetat/Heptan 1:2). Ausbeute: 170 mg.

**Beispiel 248:**

1-{2-[5-(3-Chlor-phenyl)-[1,3,4]oxadiazol-2-yl]-pyrrolidin-1-yl}-pentan-1-on

Die Stufen 1-4 entsprachen den Stufen 1-4 von Beispiel 247.

5.Stufe:

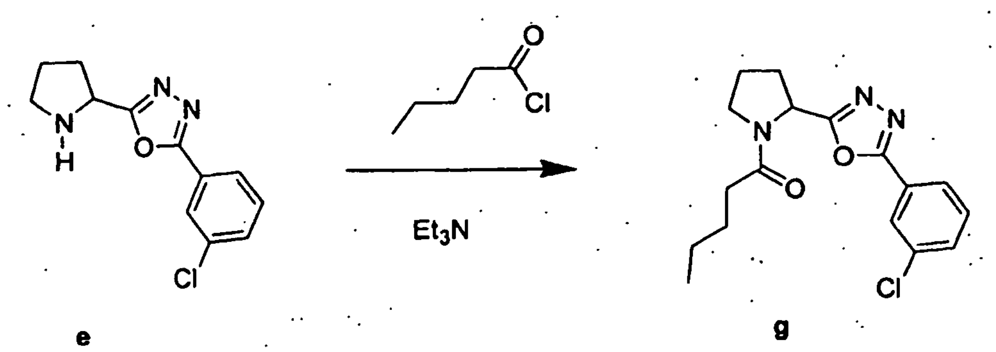


1,3 g des Oxadiazol-Derivates e wurden in 9 ml DCM gelöst. Bei einer Temperatur von - 20°C wurden 1 g Triethylamin und 620 mg 3-Valeroylchlorid in 1 ml DCM zugegeben. Das Reaktionsgemisch wurde 2 h bei - 20°C gerührt und anschließend zur Trockne eingeengt. Das Produkt wurde durch Säulenchromatographie gereinigt (Kieselgel, Ethylacetat/Heptan 1:2, danach basisches Allox, Ethylacetat/Heptan 1:2). Ausbeute: 181 mg.

**Pharmakologische Untersuchungen**

**Untersuchungen zur Serotonin-Wiederaufnahmehemmung (5HT-Uptakehemmung)**

Um diese in vitro Studien durchführen zu können, werden Synaptosomen aus Rattenhirnarealen frisch isoliert. Es findet jeweils eine sogenannte "$P_2$"-Fraktion Verwendung, die nach der Vorschrift von Gray und Whittaker (E.G. Gray und V.P. Whittaker (1962) J. Anat. 76, 79-88) präpariert wird. Für den 5HT-Uptake werden diese vesikulären Partikel aus der Medulla + Pons-Region von männlichen Rattengehirnen isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

Beispielhaft wurden die folgenden Verbindungen auf ihre 5HT-Uptakehemmung untersucht:

**Tabelle 1**

| Verbindung nach Beispiel- Nr. | 5HT Uptake, % Hemmung |
|---|---|
| 4 | 72 |
| 5 | 81 |
| 6 | 84 |
| 7 | 64 |
| 12 | 54 |

(fortgesetzt)

| Verbindung nach Beispiel- Nr. | 5HT Uptake, % Hemmung |
|---|---|
| 13 | 67 |
| 14 | 69 |
| | |
| 22 | 60 |
| 30 | 60 |
| 61 | 50 |
| 67 | 50 |
| 84 | 61 |
| 85 | 61 |
| 86 | 73 |
| 92 | 58 |
| 94 | 50 |
| 101 | 54 |
| 109 | 54 |
| 134 | 55 |
| 164 | 80 |
| 165 | 76 |
| 166 | 78 |
| 173 | 62 |
| 174 | 61 |
| 181 | 62 |
| 182 | 52 |

**Analgesieprüfung im Writhing-Test an der Maus**

Die Untersuchung auf analgetische Wirksamkeit wurde im Phenylchinon-induzierten Writhing an der Maus (modifiziert nach I.C. Hendershot und J. Forsaith (1959) J. Pharmacol. Exp. Ther. 125, 237-240) durchgeführt. Dazu wurden männliche NMRI-Mäuse im Gewicht von 25 bis 30 g verwendet. Gruppen von 10 Tieren pro Substanzdosis erhielten 10 Minuten nach intravenöser Gabe der Prüfsubstanzen 0,3 ml/Maus einer 0,02%igen wässrigen Lösung von Phenylchinon (Phenylbenzochinon, Fa. Sigma, Deisenhofen; Herstellung der Lösung unter Zusatz von 5 % Äthanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten. Alle Substanzen wurden in der Standarddosierung von 10 mg/kg getestet. Die prozentuale Hemmung (%Hemmung) der Writhingreaktion durch eine Substanz wurde nach folgender Formel berechnet:

$$\%\ \text{Hemmung} = 100 - \frac{\text{Writhingreaktionen der behandelten Tiere}}{\text{Writhingreaktionen der Kontrolltiere}} * 100$$

Für einige Substanzen wurde aus der dosisabhängigen Abnahme der Writhingreaktionen im Vergleich zu parallel untersuchten Phenylchinon-Kontrollgruppen mittels Regressionsanalyse (Auswerteprogramm Martens EDV Service, Eckental) die $ED_{50}$-Werte mit 95 % Vertrauensbereich der Writhingreaktion berechnet.

Die untersuchten erfindungsgemäßen Verbindungen zeigten eine gute analgetische Wirkung. Die Ergebnisse sind in der folgenden Tabelle zusammengefasst.

**Tabelle 2**

| Beispiel | %Hemmung der Writhingreaktion bei 10 mg/kg intravenös |
|---|---|
| 247 | 33 |
| 248 | 45 |

## Patentansprüche

**1.** Substituierte 2-Pyrrolidin-2-yl-[1,3,4]-oxadiazol - Derivate der allgemeinen Formel I,

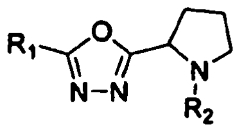

I

worin

$R_1$ Aryl oder Heteroaryl bedeutet,
$R_2$ H, $SO_2R^3$ oder $COR^4$ bedeutet;
$R^3$ und $R^4$ unabhängig voneinander $C_{1-10}$-Alkyl, $C_{3-10}$-Cycloalkyl, ($C_{1-6}$-Alkyl)- $C_{3-10}$-Cycloalkyl, Aryl, ($C_{1-6}$-Alkyl)-Aryl, Heterocyclyl, Reste von Carbonsäureestern mit 3-10 C-Atomen, Dimethylamid oder $NR^5R^6$ bedeuten,
$R^5$ und $R^6$ unabhängig voneinander H; Benzyl oder Phenyl, jeweils einfach oder mehrfach gleich oder verschieden substituiert mit F, Cl, O-Alkyl, CN, $CF_3$ oder $OCF_3$, bedeuten,
wobei der Begriff "Alkyl" acyclische, gesättigte oder ungesättigte, verzweigte oder geradkettige, unsubstituierte oder einfach oder mehrfach substituierte Kohlenwasserstoffreste umfasst, wobei auch Reste umfasst sind, deren C-Kette durch ein oder mehrere der Heteroatome N, O oder S unterbrochen ist, und der Begriff "Cycloalkyl" cyclische Kohlenwasserstoffreste umfasst, die gesättigt oder ungesättigt, unsubstituiert oder einfach oder mehrfach substituiert bzw. gegebenenfalls benzokondensiert sein können,
wobei man unter dem Begriff "substituiert" in Bezug auf "Alkyl" und "Cycloalkyl" die einfache oder mehrfache Substitution von einem oder mehreren Wasserstoffatomen durch F, Cl, Br, I, -CN, $NH_2$, NH-Alkyl, NH-Aryl, NH-Alkyl-Aryl, NH-Heterocyclyl, $N(Alkyl)_2$, $N(Alkyl\text{-}Aryl)_2$, N-Alkyl-N-Aryl, $NO_2$, OH, Ketogruppe, O-Alkyl, O-Aryl, O-Alkyl-Aryl, $C(=O)C_{1-6}$-Alkyl, C(=O)Aryl, $C(=O)C_{1-6}$-Alkyl-Aryl, C(=O)-Heterocyclyl, $CO_2H$, $CO_2$-Alkyl, $CO_2$-Alkyl-Aryl, $C(=O)NH_2$, C(=O)NH-Alkyl, C(=O)NHAryl, C(=O)NH- Heterocyclyl, $C(=O)N(Alkyl)_2$, $C(=O)N(Alkyl\text{-}Aryl)_2$, Cycloalkyl, Aryl oder Heterocyclyl, versteht.

**2.** Verbindungen gemäß Anspruch 1, **dadurch gekennzeichnet, dass**

$R_1$ Aryl oder Heteroaryl bedeutet, wobei

Aryl für Phenyl unsubstituiert oder substituiert mit F, Cl, O-Alkyl oder Phenyl

und

Heteroaryl für Pyridinyl oder Thienyl steht,

$R_2$ H, $SO_2R^3$ oder $COR^4$, wobei $R^3$ und $R^4$ unabhängig voneinander $C_{1-10}$-Alkyl, $C_{3-10}$-Cycloalkyl, ($C_{1-6}$-Alkyl)- $C_{3-10}$-Cycloalkyl; Phenyl, Benzyl oder Naphthyl unsubstituiert, einfach substituiert oder mehrfach gleich oder verschieden mit Phenyl, $NO_2$, $C_{1-6}$-Alkyl, dessen C-Atomkette ggf. durch ein oder mehrere der Heteroatome N, O oder S, vorzugsweise durch O, unterbrochen ist, O- Alkyl, $CO_2$-Alkyl, $C(=O)C_{1-6}$-Alkyl, $CH_2OC(=O)C_6H_5$, F, Cl, Br, $N(CH_3)_2$, $OCF_3$, $CF_3$, $SCHF_2$, $SCF_3$ oder $(C=O)CH_3$ ; ($C_{1-6}$-Alkyl)-Aryl, Heterocyclyl, Reste von Carbonsäureestern mit 3-10 C- Atomen, Dimethylamid oder $NR^5R^6$ bedeuten, wobei

$C_{1-10}$-Alkyl für Methyl, Propyl, Butyl, Butenyl, Isobutyl, Pentyl, Pent-3-yl, Hept-3-yl, Hept-4-yl, 2,2-Dimethylpropyl, $CH_2OCH_3$ oder $CH_2O(CH_2)_2OCH_3$, steht,
$C_{3-10}$-Cycloalkyl für Cyclopropyl, Cyclobutyl, Cyclopentyl, Adamantan- 1- yl oder 2- Phenyl- cyclopropyl steht,
($C_{1-6}$-Alkyl)-$C_{3-10}$-Cycloalkyl für $CH_2$-Cyclopentyl, $(CH_2)_2$-Cyclopentyl oder 7,7- Dimethyl- 1- methyl- bicyclo [2.2.1] heptan- 2- on steht,
($C_{1-6}$-Alkyl)-Aryl für 3,4-Dimethoxyphenyl-$CH_2$, 4-Chlorphenoxy-$CH_2$, Phenyl-CH=CH, Benzyl-$OCH_2$, Phenyl-$(CH_2)_2$, 2- Bromphenyl-$CH_2$, 1-Phenyl-propyl, 2-Chlorphenyl-CH=CH, 3-Trifluormethyl-phenyl-CH=CH, Phenoxy-$CH_2$, Phenoxy- $(CH_2)_3$ oder Phenoxy-$CH(CH_3)$ steht,
Heterocyclyl für Pyridinyl, Isoxazol, Thienyl, Furanyl, Triazol, Benzooxadiazol, Thiadiazol, Pyrazol oder Isochinolin, unsubstituiert, einfach substituiert oder mehrfach gleich oder verschieden mit Cl, $C_{1-6}$-Alkyl, Phenyl, welches wiederum unsubstituiert oder einfach oder mehrfach gleich oder verschieden mit Cl oder $C_{1-6}$-Alkyl substituiert ist, $CF_3$, $C(=O)CF_3$ oder $SCH_3$ steht,

die Reste von Carbonsäureestern mit 3-10 C-Atomen für
$CH_3OC(=O)CH_2$
$CH_3OC(=O)(CH_2)_3$
$CH_3CH_2OC(=O)CH_2$
$CH_3CH_2OC(=O)(CH_2)_2$
$CH_3C(=O)OCH_2$
$CH_3C(=O)OC(CH_3)_2$ oder
$CH_3C(=O)OCH(C_6H_5)$ stehen.

**3.** Verbindungen gemäß Anspruch 1 oder 2, **dadurch gekennzeichnet, dass**

$R_1$ Phenyl, Bisphenyl-4-yl, 3-Methoxyphenyl, 4-Chlorphenyl, 2-Fluorphenyl, Pyridin-3-yl, Pyridin-4-yl oder Thiophen-2-yl bedeutet,
$R_2$ H, $SO_2R^3$ oder $COR^4$ bedeutet, wobei
$R^3$ und $R^4$ unabhängig voneinander $CH_3CH_2OCO(CH_2)_2$-, 2,4-Dimethoxy- phenyl, 2-Chlor-pyridin-3-yl, 2-Chlor-pyridin-4-yl, 7,7-Dimethyl-1-methyl- bicyclo[2.2.1]heptan-2-on, 3-Dimethyl-amino-phenyl, 3,4-Dimethoxy- phenyl, 2,5-Dimethoxy-phenyl, 4-Chlor-phenyl, 4,5-Dichlor-thiophen-2- yl, 2,4,6-Trimethylphenyl, 4-Chlor-phenoxy-methyl, $C_6H_5CH=CH$-, 5-Methyl-2-phenyl-2H-[1,2,3]triazol-4-yl, 2,4-Difluorphenyl, 2,6-Difluorphenyl, 4-Bromphenyl, 4-Ethoxy-phenyl, 4-Trifluormethoxy- phenyl, 2,5-Bis-trifluormethyl-phenyl, 1-Phenyl-5-propyl-1H-pyrazol-4-yl, 3-Methoxy-phenyl, 2-Methyl-6-trifluormethyl-pyridin-3-yl, 4- Trifuormethylsulfanyl-phenyl, 2,2,2-Trifluor-1-3,4-dihydro-1H- isochinolin-2-yl-ethanon oder $NR^5R^6$ bedeuten, wobei
$R^5$ und $R^6$ unabhängig voneinander H oder 3-Methoxyphenyl, 4-Methoxyphenyl, 4-Ethoxyphenyl, 2,5-Dimethoxy-phenyl, 2,5-Dichlorphenyl, 2,5-Difluorphenyl, 4-Fluorbenzyl, 4-Chlor-3- trifluormethyl-phenyl, 4-Trifuormethoxy-phenyl oder 3-Cyanophenyl bedeuten.

**4.** Ein substituiertes 2-Pyrrolidin-2-yl-[1,3,4]-oxadiazol - Derivat gemäß Anspruch 1 aus der Gruppe umfassend:

4-Oxo-4-[2-(5-pyridin-4-yl-[1,3,4]oxadiazol-2-yl)-pyrrolidin-1-yl]-buttersäureethylester
(2,4-Dimethoxy-phenyl)-[2-(5-pyridin-4-yl-[1,3,4]oxadiazol-2-yl)-pyrrolidin-1-yl]-methanon
(2-Chlor-pyridin-3-yl)-[2-(5-pyridin-4-yl-[1,3,4]oxadiazol-2-yl)-pyrrolidin-1-yl]-methanon
7,7-Dimethyl-1-[2-(5-pyridin-4-yl-[1,3,4]oxadiazol-2-yl)-pyrrolidin-1-sulfonylmethyl]-bicyclo[2.2.1]heptan-2-on
(3-Dimethylamino-phenyl)-[2-(5-pyridin-3-yl-[1,3,4]oxadiazol-2-yl)-pyrrolidin-1-yl]-methanon
2-(3,4-Dimethoxy-phenyl)-1-[2-(5-pyridin-4-yl-[1,3,4]oxadiazol-2-yl)-pyrrolidin-1-yl]-ethanon
[2-(5-Biphenyl-4-yl-[1,3,4]oxadiazol-2-yl)-pyrrolidin-1-yl]-(4-chlor-phenyl)-methanon
4-{5-[1-(4,5-Dichlor-thiophene-2-sulfonyl)-pyrrolidin-2-yl]-[1,3,4]oxadiazol-2-yl}-pyridin
3-{5-[1-(2,5-Dimethoxy-benzylsulfonyl)-pyrrolidin-2-yl]-[1,3,4]oxadiazol-2-yl}-pyridin
2-(2-Fluor-phenyl)-5-[1-(2,4,6-trimethyl-benzylsulfonyl)-pyrrolidin-2-yl]-[1,3,4]oxadiazol
2-(4-Chlor-phenoxy)-1-[2-(5-pyridin-4-yl-[1,3,4]oxadiazol-2-yl)-pyrrolidin-1-yl]-ethanon
3-Phenyl-1-[2-(5-phenyl-[1,3,4]oxadiazol-2-yl)-pyrrolidin-1-yl]-propenon

## Claims

**1.** Substituted 2-pyrrolidin-2-yl-[1,3,4]-oxadiol derivatives with the general formula I

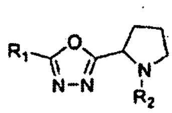

I

wherein
$R_1$ means aryl or heteroaryl,
$R_2$ means H, $SO_2R^3$ or $COR^4$,
$R^3$ and $R^4$, independently of one another, mean $C_{1-10}$-alkyl, $C_{3-10}$-cycloalkyl, ($C_{1-6}$-alkyl)-$C_{3-10}$-cycloalkyl, aryl, ($C_{1-6}$-alkyl)-aryl, heterocyclyl, carboxylate residues with 3-10 C atoms, dimethylamide or $NR^5R^6$,
$R^5$ and $R^6$, independently of one another, mean H; benzyl or phenyl, respectively mono- or polysubstituted, with the same or different substituents, with F, Cl, O-alkyl, CN, $CF_3$ or $OCF_3$,
wherein the term "alkyl" covers acyclic, saturated or unsaturated, branched or straight-chain, unsubstituted or mono- or polysubstituted hydrocarbon residues, wherein residues are also covered, the C chain of which is interrupted by one or more of the heteroatoms N, O or S, and the term "cycloalkyl" covers cyclic hydrocarbon residues, which can be saturated or unsaturated, unsubstituted or mono- or polysubstituted or optionally benzo-condensed.
wherein the term "substituted" with respect to "alkyl" and "cycloalkyl" is understood to mean the mono- or polysubstitution of one or more hydrogen atoms by F, Cl, Br, I, -CN, $NH_2$, NH-alkyl, NH-aryl, NH-alkyl-aryl, NH-heterocyclyl, $N(alkyl)_2$, $N(alkyl-aryl)_2$, N-alkyl-N-aryl, $NO_2$, OH, keto group, O-alkyl, O-aryl, O-alkyl-aryl, $C(=O)C_{1-6}$-alkyl, C(=O) aryl, $C(=O)C_{1-6}$-alkyl-aryl, C(=O)heterocyclyl, $CO_2H$, $CO_2$-alkyl, $CO_2$-alkyl-aryl, $C(=O)NH_2$, C(=O)NH-alkyl, C(=O) NHaryl, C(=O)NH-heterocyclyl, $C(=O)N(alkyl)_2$, $C(=O)N(alkyl-aryl)_2$, cycloalkyl, aryl or heterocyclyl.

**2.** Compounds according to claim 1, **characterised in that**
$R_1$ means aryl or heteroaryl, wherein
aryl stands for phenyl that is unsubstituted or substituted with F, Cl, O-alkyl or phenyl and heteroaryl stands for pyridinyl or thienyl,
$R_2$ means H, $SO_2R^3$ or $COR^4$, wherein
$R^3$ and $R^4$, independently of one another, mean $C_{1-10}$-alkyl, $C_{3-10}$-cycloalkyl, ($C_{1-6}$-alkyl)-$C_{3-10}$-cycloalkyl; phenyl, benzyl or naphthyl that is unsubstituted, mono-substituted or polysubstituted, with the same or different substituents, with phenyl, $NO_2$, $C_{1-6}$-alkyl, the C atom chain of which in possibly interrupted by one or more of heteroatoms, N, O or S, preferably by O, O-alkyl, $CO_2$-alkyl, $C(=O)C_{1-6}$-alkyl, $CH_2OC(=O)C_6H_5$, F, Cl, Br, $N(CH_3)_2$, $OCF_3$, $CF_3$, $SCHF_2$, $SCF_3$ or $(C=O)CH_3$; ($C_{1-6}$-alkyl)-aryl, heterocyclyl, residues of carboxylates with 3-10 C atoms, dimethylamide or $NR^5R^6$,
wherein
$C_{1-10}$-alkyl stands for methyl, propyl, butyl, butenyl, isobutyl, pentyl, pent-3-yl, hept-3-yl, hept-4-yl, 2,2-dimethylpropyl, $CH_2OCH_3$ or $CH_2O(CH_2)_2OCH_3$,
$C_{3-10}$-cycloalkyl stands for cyclopropyl, cyclobutyl, cyclopentyl, adamantan-1-yl or 2-phenyl-cyclopropyl,
($C_{1-6}$-alkyl)-$C_{3-10}$-cycloalkyl stands for $CH_2$-cyclopentyl, $(CH_2)_2$-cyclopentyl or 7,7-dimethyl-1-methyl-bicyclo[2.2.1]

heptan-2-one,
($C_{1-6}$-alkyl)-aryl stands for 3,4-dimethoxyphenyl-$CH_2$, 4-chlorophenoxy-$CH_2$, phenyl-CH=CH, benzyl-$OCH_2$, phenyl-$(CH_2)_2$, 2-bromophenyl-$CH_2$, 1-phenyl-propyl, 2-chlorophenyl-CH=CH, 3-trifluoromethyl-phenyl-CH=CH, phenoxy-$CH_2$, phenoxy-$(CH_2)_3$ or phenoxy-$CH(CH_3)$,
heterocyclyl stands for pyridinyl, isoxazole, thienyl, furanyl, triazole, benzo-oxadiazole, thiadiazole, pyrazole or isoquinoline, unsubstituted, mono-substituted or polysubstituted, with the same or different substituents, with Cl, $C_{1-6}$-alkyl, phenyl, which is in turn unsubstituted or mono- or polysubstituted, with the same or different substituents, with Cl or $C_{1-6}$-alkyl, $CF_3$, $C(=O)CF_3$ or $SCH_3$,
the residues of carboxylates with 3-10 C atoms stand for
$CH_3OC(=O)CH_2$
$CH_3OC(=O)(CH_2)_3$
$CH_3CH_2OC(=O)CH_2$
$CH_3CH_2OC(=O)(CH_2)_2$
$CH_3C(=O)OCH_2$
$CH_3C(=O)OC(CH_3)_2$ or
$CH_3C(=O)OCH(C_6H_5)$.

**3.** Compounds according to claim 1 or 2, **characterised in that**
$R_1$ means phenyl, bisphenyl-4-yl, 3-methoxyphenyl, 4-chlorophenyl, 2-fluorophenyl, pyridin-3-yl, pyridin-4-yl or thiophen-2-yl,
$R_2$ means H, $SO_2R^3$ or $COR^4$, wherein
$R^3$ and $R^4$, independently of one another, mean $CH_3CH_2OCO(CH_2)_2$-, 2,4-dimethoxy-phenyl, 2-chloro-pyridin-3-yl, 2-chloro-pyridin-4-yl, 7,7-dimethyl-1-methyl-bicyclo[2.2.1]hepan-2-one, 3-dimethyl-amino-phenyl, 3,4-dimethoxy-phenyl, 2.5-dimethoxy-phenyl, 4-chloro-phenyl, 4.5-dichloro-thiophen-2-yl, 2,4,6-trimethylphenyl, 4-chloro-phenoxy-methyl, $C_6H_5CH$=CH-, 5-methyl-2-phenyl-2H-[1,2,3]triazol-4-yl, 2,4-difluorophenyl, 2,6-difluorophenyl, 4-bromo-phenyl, 4-ethoxy-phenyl, 4-trifluoromethoxy-phenyl, 2,5-bis-trifluoromethyl-phenyl, 1-phenyl-5-propyl-1H-pyrazol-4-yl, 3-methoxy-phenyl, 2-methyl-6-trifluoromethyl-pyridin-3-yl, 4-trifluoromethylsulphanyl-phenyl, 2,2,2-trifluoro-1-3,4-dihydro-1H-isoquinolin-2-yl-ethanone or $NR^5R^6$, wherein
$R^5$ and $R^6$, independently of one another, mean H or 3-methoxyphenyl, 4-methoxyphenyl, 4-ethoxyphenyl, 2,5-dimethoxy-phenyl, 2,5-dichlorophenyl, 2,5-difluorophenyl, 4-fluorobenzyl, 4-chloro-3-trifluoromethyl-phenyl, 4-trifluoromethoxy-phenyl or 3-cyanophenyl.

**4.** A substituted 2-pyrrolidin-2-yl-[1,3,4]-oxadiazole derivative according to claim 1 from the group comprising:

4-oxo-4-[2-(5-pyridin-4-yl-[1,3,4]oxadiazol-2-yl)-pyrrolidin-1-yl] ethyl butyrate
(2,4-dimethoxy-phenyl)-[2-(5-pyridin-4-yl-[1,3,4]oxadiazol-2-yl)-pyrrolidin-1-yl] methanone
(2-chloro-pyridin-3-yl)-[2-(5-pyridiin-4-yl-[1,3,4]oxadiazol-2-yl)-pyrrolidin-1-yl] methanone
7,7-dimethyl-1-[2-(5-pyridin-4-yl-[1,3,4]oxadiazol-2-yl)-pyrrolidin-1-sulphonylmethyl]-bicyclo[2.2.1]heptan-2-one
(3-dimethylamino-phenyl)-[2-(5-pyridin-3-yl-[1,3,4]oxadiazol-2-yl)-pyrrolidin-1-yl] methanone
2-(3,4-dimethoxy-phenyl)-1-[2-(5-pyridin-4-yl-[1,3,4]oxadiazol-2-yl)-pyrrolidin-1-yl] ethanone
[2-{5-biphenyl-4-yl-[1,3,4]oxadiazol-2-yl)-pyrrolidin-1-yl]-(4-chloro-phenyl} methanone
4- {5-[1-(4,5-dichloro-thiophene-2-sulphonyl)-pyrrolidin-2-yl]-[1,3,4]oxadiazol-2-yl} pyridine
3-(5-[1-(2,5-dimethoxy-benzylsulphonyl)-pyrrolidin-2-yl]-[1,3,4]oxadiazol-2-yl) pyridine
2-(2-fluoro-phenyl)-5-[1-(2,4,6-trimethyl-benzylsulphonyl)-pyrrolidin-2-yl]-[1,3,4]oxadiazole
2-(4-chloro-phenoxy)-1-[2-(5-pyridin-4-yl-[1,3,4]oxadiazol-2-yl)-pyrrolidin-1-yl] ethanone
3-phenyl-1-[2-(5-phenyl-[1,3,4]oxadiazol-2-yl)-pyrrolidin-1-yl] propenone

## Revendications

**1.** Dérivés substitués de 2-pyrrolidin-2-yl[1,3,4]-oxadiazole de formule générale I,

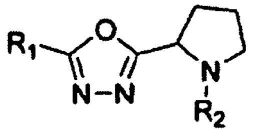

I

où

$R_1$ signifie aryle ou hétéroaryle,
$R_2$ signifie H, $SO_2R^3$ ou $COR^4$ ;
$R^3$ et $R^4$ signifient, indépendamment l'un de l'autre, $C_{1-10}$-alkyle, $C_{3-10}$-cycloalkyle, ($C_{1-6}$-alkyl) -$C_{3-10}$- cycloalkyle, aryle, ($C_{1-6}$-alkyl)-aryle, hétérocyclyle, des radicaux d'esters d'acides carboxyliques comprenant 3-10 atomes de carbone, diméthylamide ou $NR^5R^6$,
$R^5$ et $R^6$ signifient, indépendamment l'un de l'autre, H ; benzyle ou phényle, à chaque fois monosubstitué ou polysubstitué, de manière identique ou différente, par F, Cl, O-alkyle, CN, $CF_3$ ou $OCF_3$,
où le concept "alkyle" comprend des radicaux hydrocarbonés acycliques, saturés ou insaturés, ramifiés ou linéaires non substitués ou monosubstitués ou polysubstitués, comprenant également des radicaux dont la chaîne carbonée est interrompue par un ou plusieurs des hétéroatomes N, O ou S, et le concept "cycloalkyle" comprend des radicaux hydrocarbonés cycliques, qui peuvent être saturés ou insaturés, non substitués ou monosubstitués ou polysubstitués ou le cas échéant benzocondensés,
où on entend par le concept "substitué" par rapport à "alkyle" et "cycloalkyle" la substitution simple ou multiple d'un ou de plusieurs atomes d'hydrogène par F, Cl, Br, I, -CN, $NH_2$, NH-alkyle, NH-aryle, NH-alkyl-aryle, NH-hétérocyclyle, $N(alkyle)_2$, $N(alkyl\text{-}aryle)_2$, N-alkyl-N-aryle, $NO_2$, OH, groupe céto, O-alkyle, O-aryle, O-alkyl-aryle, C(=O)$C_{1-6}$-alkyle, C(=O)aryle, C(=O)$C_{1-6}$-alkyl-aryle, C(=O)-hétérocyclyle, $CO_2H$, $CO_2$-alkyle, $CO_2$-alkyl-aryle, C(=O)$NH_2$, C(=O)NH-alkyle, C(=O)NH-aryle, C(=O)NH-hétérocyclyle, C(=O)N$(alkyle)_2$, C(=O)N$(alkyl\text{-}aryle)_2$, cycloalkyle, aryle ou hétérocyclyle.

2. Composés selon la revendication 1, **caractérisés en ce que**

$R_1$ signifie aryle ou hétéroaryle, où aryle représente phényle, non substitué ou substitué par F, Cl, O-alkyle ou phényle et hétéroaryle représente pyridinyle ou thiényle,
$R_2$ signifie H, $SO_2R^3$ ou $COR^4$, où
$R^3$ et $R^4$ signifient, indépendamment l' un de l'autre, $C_{1-10}$-alkyle, $C_{3-10}$-cycloalkyle, ($C_{1-6}$-alkyl) -$C_{3-10}$-cycloalkyle ; phényle, benzyle ou naphtyle, non substitué, monosubstitué ou polysubstitué, de manière identique ou différente, par phényle, $NO_2$, $C_{1-6}$-alkyle, dont la chaîne d'atomes de carbone est le cas échéant interrompue par un ou plusieurs des hétéroatomes N, O ou S, de préférence par O, O- alkyle, $CO_2$-alkyle, C(=O)$C_{1-6}$-alkyle, $CH_2OC(=O)C_6H_6$, F, Cl, Br, $N(CH_3)_2$, $OCF_3$, $CF_3$, $SCHF_2$, $SCF_3$ ou $(C{=}O)CH_3$ ; ($C_{1-6}$-alkyl)-aryle, hétérocyclyle, des radicaux d'esters d'acides carboxyliques comprenant 3-10 atomes de carbone, diméthylamide ou $NR^5R^6$, où
$C_{1-10}$-alkyle représente méthyle, propyle, butyle, butényle, isobutyle, pentyle, pent-3-yle, hept-3-yle, hept-4-yle, 2,2-diméthylpropyle, $CH_2OCH_3$ ou $CH_2O(CH_2)_2OCH_3$,
$C_{3-10}$-cycloalkyle représente cyclopropyle, cyclobutyle, cyclopentyle, adamantan-1-yle ou 2-phénylcyclopropyle,
($C_{1-6}$-alkyl) -$C_{3-10}$-cycloalkyle représente $CH_2$- cyclopentyle, $(CH_2)_2$-cyclopentyle ou 7,7- diméthyl-1-méthylbicyclo[2,2,1]heptan-2-one,
($C_{1-6}$-alkyl)-aryle représente 3,4-diméthoxyphényl- $CH_2$, 4-chlorophénoxy-$CH_2$, phényl-CH=CH, benzyl-$OCH_2$, phényl-$(CH_2)_2$, 2-bromophényl-$CH_2$, 1-phénylpropyle, 2-chlorophényl-CH=CH, 3- trifluorométhylphényl-CH=CH, phénoxy-$CH_2$, phénoxy-$(CH_2)_3$ ou phénoxy-$CH(CH_3)$,
hétérocyclyle représente pyridinyle, isoxazole, thiényle, furannyle, triazole, benzoxadiazole, thiadiazole, pyrazole ou isoquinoléine, non substitué, monosubstitué ou polysubstitué, de manière identique ou différente, par Cl, $C_{1-6}$-alkyle, phényle - qui est à son tour non substitué ou monosubstitué ou polysubstitué, de manière identique ou différente, par Cl ou $C_{1-6}$-alkyle, $CF_3$, C(=O)$CF_3$ ou $SCH_3$,

les radicaux d'esters d'acide carboxylique comprenant 3-10 atomes de carbone représentent
$CH_3OC(=O)CH_2$
$CH_3OC(=O)(CH_2)_3$
$CH_3CH_2OC(=O)CH_2$

$CH_3CH_2OC (=O)(CH_2)_2$
$CH_3C(=O)OCH_2$
$CH_3C(=O)OC (CH_3)_2$ ou
$CH_3C(=O)OCH(C_6H_5)$.

**3.** Composés selon la revendication 1 ou 2, **caractérisés en ce que**

$R_1$ signifie phényle, biphényl-4-yle, 3- méthoxyphényle, 4-chlorophényle, 2-fluorophényle, pyridin-3-yle, pyridin-4-yle ou thiophén-2-yle,
$R_2$ signifie H, $SO_2R^3$ ou $COR^4$, où
$R^3$ et $R^4$ signifient, indépendamment l'un de l'autre, $CH_3CH_2OCO(CH_2)_2$-, 2,4-diméthoxyphényle, 2- chloropyridin-3-yle, 2-chloropyridin-4-yle, 7,7- diméthyl-1-méthylbicyclo[2,2,1]heptan-2-one, 3- diméthylaminophényle, 3,4-diméthoxyphényle, 2,5- diméthoxyphényle, 4-chlorophényle, 4,5- dichlorothiophén-2-yle, 2,4,6-triméthylphényle, 4- chlorophénoxyméthyle, $C_6H_5CH$=CH-, 5-méthyl-2- phényl-2H-[1,2,3]triazol-4-yle, 2,4- difluorophényle, 2,6-difluorophényle, 4- bromophényle, 4-éthoxyphényle, 4- trifluorométhoxyphényle, 2,5- bistrifluorométhylphényle, 1-phényl-5-propyl-1H- pyrazol-4-yle, 3-méthoxyphényle, 2-méthyl-6- trifluorométhylpyridin-3-yle, 4- trifuorométhylsulfanylphényle, 2,2,2-trifluoro-1- 3,4-dihydro-1H-isoquinoléin-2-yléthanone ou $NR^5R^6$, où
$R^5$ et $R^6$ signifient indépendamment l'un de l'autre H ou 3-méthoxyphényle, 4-méthoxyphényle, 4- éthoxyphényle, 2,5-diméthoxyphényle, 2,5- dichlorophényle, 2,5-difluorophényle, 4- fluorobenzyle, 4-chloro-3-trifluorométhylphényle, 4-trifluorométhoxyphényle ou 3-cyanophényle.

**4.** Dérivé substitué de 2-pyrrolidin-2-yl-[1,3,4]-oxadiazole selon la revendication 1 du groupe comprenant :

- l'ester éthylique de l'acide 4-oxo-4-[2-(5-pyridin-4-yl-[1,3,4]oxadiazol-2-yl)-pyrrolidin-1-yl]-butyrique
- la (2,4-diméthoxyphényl)-[2-(5-pyridin-4-yl-[1,3,4]oxadiazol-2-yl)-pyrrolidin-1-yl]-méthanone
- la (2-chloropyridin-3-yl)-[2-(5-pyridin-4-yl-[1,3,4]oxadiazol-2-yl)-pyrrolidin-1-yl]-méthanone
- la 7,7-diméthyl-1-[2-(5-pyridin-4-yl-[1,3,4]oxadiazol-2-yl)-pyrrolidin-1-sulfonylméthyl]-bicyclo[2,2,1]heptan-2-one
- (3-diméthylamino-phényl)-[2-(5-pyridin-3-yl-[1,3,4]oxadiazol-2-yl)-pyrrolidin-1-yl]-méthanone
- 2-(3,4-diméthoxyphényl)-1-[2-(5-pyridin-4-yl-[1,3,4]oxadiazol-2-yl)-pyrrolidin-1-yl]-éthanone
- [2-(5-biphényl-4-yl-[1,3,4]oxadiazol-2-yl)-pyrrolidin-1-yl]-(4-chlorophényl)-méthanone
- 4-{5-[1-(4,5-dichloro-thiophène-2-sulfonyl)-pyrrolidin-2-yl]-[1,3,4]oxadiazol-2-yl}-pyridine
- 3-{5-[1-(2,5-diméthoxy-benzylsulfonyl)-pyrrolidin-2-yl]-[1,3,4]oxadiazol-2-yl}-pyridine
- 2-(2-fluoro-phényl)-5-[1-(2,4,6-triméthyl-benzylsulfonyl)-pyrrolidin-2-yl]-[1,3,4]oxadiazole
- 2-(4-chloro-phénoxy)-1-[2-(5-pyridin-4-yl-[1,3,4]oxadiazol-2-yl)-pyrrolidin-1-yl]-éthanone
- 3-phényl-1-[2-(5-phényl-[1,3,4]oxadiazol-2-yl)-pyrrolidin-1-yl]-propénone.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0104116 A
- JP 2001247569 B
- US 3912747 A


### In der Beschreibung aufgeführte Nicht-Patentliteratur


- **van Schayck et al.** *MMP,* 1998, 304-313
- **Jung et al.** *J. Gen. Intern. Med.,* 1997, vol. 12/6, 384-389
- **Onghena ; Van Houdenhove.** *Pain,* 1992, vol. 49, 205-219
- **Feuerstein.** *Der Schmerz,* 1997, vol. 11, 213-226
- **Rowbotham.** *The Pain Medicine Journal Club,* 1997, vol. 3/3, 119-122
- **Berard.** *Int. Med. J.,* 1996, vol. 3/4, 257-259
- **Borg et al.** *J. Org. Chem.,* 1995, vol. 60, 3112-3120
- **Brain et al.** *Synlett,* 2001, 382-384
- Remington’s Pharmaceutical Sciences. Mack Publishing Company, 1985
- **E.G. Gray ; V.P. Whittaker.** *J. Anat.,* 1962, vol. 76, 79-88
- **M.Ch. Frink ; H.-H. Hennies ; W. Englberger ; M. Haurand ; B. Wilffert.** *Arzneim.-Forsch./Drug Res.,* 1996, vol. 46 (III), 11, 1029-1036
- **I.C. Hendershot ; J. Forsaith.** *J. Pharmacol. Exp. Ther.,* 1959, vol. 125, 237-240